# FASCICULE DE BREVET EUROPEEN

(11) **EP 1 232 395 B1**
(45) Date de publication et mention de la délivrance du brevet: **10.01.2007**
(21) Numéro de dépôt: 00979723.4
(22) Date de dépôt: 13.11.2000
(51) Int. Cl.: G01N 33/68

(54) **PROCEDE DE DIAGNOSTIC D'UNE ESST PROVOQUEE PAR UNE SOUCHE D'ATNC DANS UN ECHANTILLON BIOLOGIQUE**
VERFAHREN ZUR DIAGNOSE EINER VON EINEM PRIONENSTAMM VERURSACHTEN ÜBERTRAGBAREN SUBAKUTEN SPONGIFORMEN ENZEPHALOPATHIE IN EINER BIOLOGISCHEN PROBE
METHOD FOR DIAGNOSING A TRANSMISSIBLE SPONGIFORM SUBACUTE ENCEPHALOPATHY CAUSED BY AN UNCONVENTIONAL TRANSMISSIBLE AGENT STRAIN IN A BIOLOGICAL SAMPLE

(30) Priorité: 12.11.1999 FR 9914242
(43) Date de publication de la demande: 21.08.2002
(73) Titulaire: COMMISSARIAT A L'ENERGIE ATOMIQUE, 75015 Paris (FR)
(72) Inventeur: DESLYS, Jean-Philippe, F-78150 Le Chesnay (FR); COMOY, Emmanuel, F-91190 SAINT-AUBIN (FR); GRASSI, Jacques, F-91440 Bures-sur-Yvette (FR)
(74) Mandataire: Vialle-Presles, Marie José
(86) Numéro de dépôt international: PCT/FR2000/003159
(87) Numéro de publication internationale: WO 2001/035104

(56) Documents cités:
- EP-A- 0 861 900
- WO-A-00/22438
- WO-A-00/26238
- WO-A-00/29849
- WO-A-00/29850
- WO-A-98/30909
- WO-A-99/41280
- WO-A-99/66956
- PATENT ABSTRACTS OF JAPAN vol. 1999, no. 01, 29 janvier 1999 (1999-01-29) & JP 10 267928 A (NORIN SUISANSYO KACHIKU EISEI SHIKENJO;NIPPI:KK), 9 octobre 1998 (1998-10-09)
- MEYER RUDOLF K ET AL: "Detection of bovine spongiform encephalopathy-specific PrPSc by treatment with heat and guanidine thiocyanate." JOURNAL OF VIROLOGY, vol. 73, no. 11, novembre 1999 (1999-11), pages 9386-9392, XP000911755 ISSN: 0022-538X
- HORNEMANN SIMONE ET AL: "A scrapie-like unfolding intermediate of the prion protein domain PrP(121-231) induced by acidic pH." PROCEEDINGS OF THE NATIONAL ACADEMY OF SCIENCES OF THE UNITED STATES, vol. 95, no. 11, 26 mai 1998 (1998-05-26), pages 6010-6014, XP002137801 May 26, 1998 ISSN: 0027-8424
- KRASEMANN S ET AL: "Induction of antibodies against human prion proteins (PrP) by DNA-mediated immunization of PrPmice" JOURNAL OF IMMUNOLOGICAL METHODS,NL,ELSEVIER SCIENCE PUBLISHERS B.V.,AMSTERDAM, vol. 199, no. 2, 15 décembre 1996 (1996-12-15), pages 109-118, XP004071808 ISSN: 0022-1759
- 'Merck Index', Entry: Octoxynol
- 'Merck Index', Entry: Polysorbate 80
- "Soltec Ventures Online catelogue" Extrait de l'Internet: URL:http://www.soltecventures.com/webdatab ases_interface/biocatxi/results_page.asp> [extrait le 2004-01-20]
- HARRIS; ANGAL: 'Protein Purification Methods', 1995, IRL PRESS * page 295 - page 297 *
- "Zymed Laboratories Inc. catalogue" 8 mai 2003 (2003-05-08), Catalogue ref: 10-1141
- "Affiland Online Catalgue" Extrait de l'Internet: URL:http://www.affiland.com/affinity/prote inarff.htm> [extrait le 2004-01-20] product code: r-PRA36-4FF

## Description

La présente invention est relative à un procédé de diagnostic d'une ESST provoquée par une souche d'ATNC dans un échantillon biologique par détection de la PrPres ainsi qu'à son utilisation dans le cadre d'un diagnostic différentiel des différentes souches d'ATNC dans un échantillon biologique.

Les encéphalopathies subaiguës spongiformes transmissibles (ESST) sont provoquées par des agents transmissibles non conventionnels (ATNC), encore appelés prions, dont la nature précise reste contestée à ce jour. Les ESST comprennent essentiellement la maladie de Creutzfeldt-Jakob, chez l'homme (MCJ ou CJD pour *Creutzfeldt-Jakob disease*), la tremblante chez le mouton et la chèvre et l'encéphalopathie spongiforme bovine (ESB ou BSE pour *bovine spongiform encephalopathy*) chez lez bovins ; d'autres encéphalopathies ont été mises en évidence chez les félidés, chez le vison ou certains animaux sauvages, tels que le cerf ou l'élan.

Ces maladies sont d'évolution constamment fatale et il n'existe, à l'heure actuelle, aucun traitement efficace.

Dans les ESST, il existe une accumulation d'une protéine de l'hôte, la PrP (ou protéine du prion), sous une forme anormale (PrPres), principalement dans le système nerveux central ; la PrPres copurifie avec l'infectiosité et son accumulation précède l'apparition des lésions histologiques. *In vitro,* elle est toxique pour des cultures de neurones.

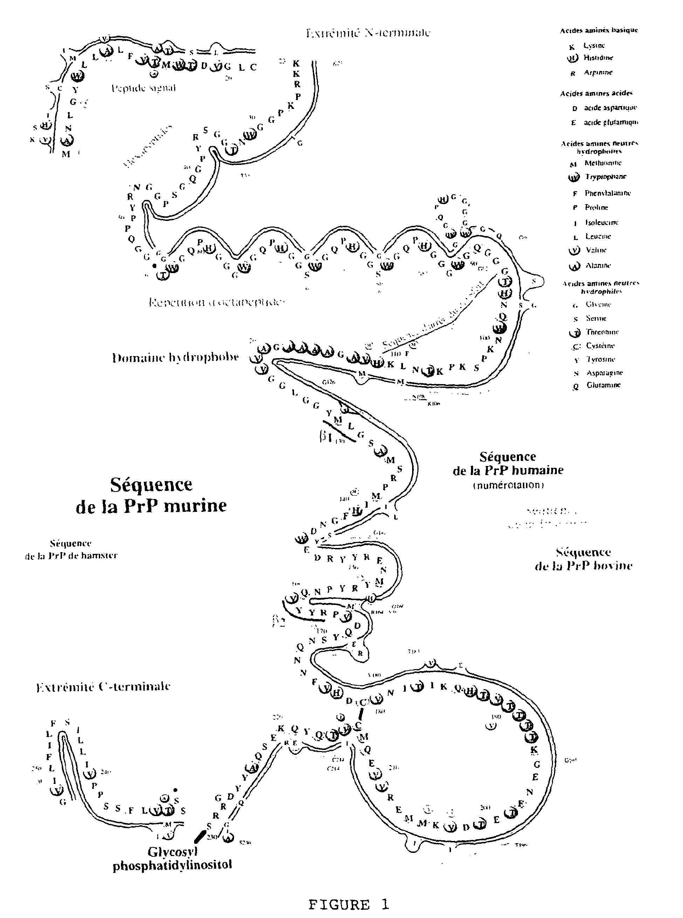

Les deux isoformes de la PrP présentent la même séquence en acides aminés (voir figure 1), mais diffèrent dans leur structure secondaire : la PrPres présente un contenu significativement plus élevé en feuillets β plissés, alors que la PrP normale (PrPsens) présente un plus grand nombre d'hélices α.

Deux propriétés biochimiques permettent, généralement, de distinguer ces deux isoformes.
- la PrPres est partiellement résistante aux protéases, notamment à la protéinase K (PK), qui entraîne un clivage de son extrémité N-terminale. Après action de la PK, la PrPres est souvent appelée PrP27-30 à cause du poids moléculaire apparent de la forme biglycosylée ; il est généralement admis que le site de clivage de la PrPres se situe entre les acides aminés 89 et 90 (Prusiner et al, Cell, 1984) pour les souches habituelles.
- la PrPres est insoluble dans les détergents non-ioniques, tels que le Triton X100 ou le Triton 114.

La forme normale de la protéine du prion (PrPsens) est en principe complètement dégradée par les protéases et est parfaitement soluble en présence de détergents non-ioniques.

Les séquences peptidiques de PrPsens de hamster, humaine, bovine et ovine sont représentées à la figure 1 ; elles comprennent notamment un motif octa-peptide (P(H/Q)GGG(-/T)WGQ), répété 4 ou 5 fois, selon les espèces. Ce motif octa-peptide répété correspond, aux acides aminés 51-91 de la PrP (numérotation de la séquence de la PrP humaine) (B. Oesch et al., 1991).

Pour déceler la présence de l'agent infectieux, les méthodes les plus récentes sont fondées sur une détection sélective de la PrP anormale (PrPres), liée à l'agent infectieux, en tirant profit de sa résistance partielle aux protéases.

On peut distinguer :
- les méthodes de Western-blot qui reposent sur la détection immunologique de la PrPres dans un extrait de tissus, après traitement de l'extrait par une protéase de façon à détruire l'isoforme normale de la PrP (PrPsens), séparation des protéines de l'extrait par électrophorèse, transfert sur une membrane de polymère, et détection par un anticorps spécifique reconnaissant la PrP (Schaller O. et al., 1999),
- les tests de type ELISA, qui font également intervenir un traitement des extraits de tissus par une protéase.

Parmi ces différents tests, qui font intervenir un traitement des extraits de tissus par une protéase, on peut citer :
- celui décrit par Serban et al. (Neurology, 1990, **40**, 110), qui ont développé un test de détection de la PrPres qui inclut l'immobilisation des protéines sur une membrane de nitrocellulose, suivie d'une digestion protéasique, d'une dénaturation et d'une immunodétection avec des anticorps monoclonaux.
- celui décrit par Oesch et al. (Biochemistry, 1994, **33**, 5926-5931), qui ont proposé, pour quantifier la quantité de PrPres, un test d'immunofiltration pour la purification de la PrPres (ELIFA ou *enzyme-linked immunofiltration assay*)*.*
- celui décrit par Gratwohl et al., 1997, qui proposent un dosage de type ELISA. Après traitement des échantillons à la protéinase K et purification de la PrPres par centrifugation, celle-ci est adsorbée sur des plaques de microtitration et détectée à l'aide d'anticorps polyclonaux de lapin.
- celui décrit par Safar et al., 1998, qui n'utilise pas la protéinase K et compare l'immunoréactivité de la PrPres immobilisée sur un support solide selon qu'elle a été soumise ou non à un traitement dénaturant.

De manière générale, ces différents tests présentent l'inconvénient de manquer de sensibilité et ainsi d'entraîner des faux-négatifs.

D'autres méthodes proposent des traitements de l'échantillon par des produits dénaturants (Oesch et al., 1994 et 1999 ; WO 00/22438, The Regents of the University of California), un traitement limité à la protéinase K (WO 00/29850, Wallac Oy et al.) ou un traitement par une métallopeptidase (WO 00/22438) qui rendent accessibles des sites antigéniques cachés, détectables par l'anticorps monoclonal 3F4 qui reconnaît la région 109-112 de la PrP (WO 00/29850 ou WO 00/22438).

La méthode, décrite dans WO 00/29850, Wallac Oy et al., présente l'inconvénient majeur de manquer de spécificité, en raison notamment d'une élimination incomplète de la PrPsens, dans les conditions de traitement préconisées, alors que la méthode décrite dans WO 00/22438, The Regents of the University of Califomia manque de sensibilité, en raison de l'utilisation de l'anticorps de révélation 3F4 (WO 00/22438), qui ne se lie qu'à un seul motif sur la protéine.

Le Demandeur, a proposé, récemment un test pour la détection quantitative de la PrPres, qui comprend une étape de purification qui conduit à une détection significativement plus sensible et qui représente une grande avancée pour la surveillance sanitaire et le dosage de la PrPres dans les abattoirs. Ce procédé est notamment décrit dans la Demande Internationale PCT WO 99/41280 et dans un rapport préliminaire de la Direction Générale XXIV de la Commission Européenne (Politique des consommateurs et protection de leur santé ; http://europa.eu.int/comm/dg24/health/).

Toutefois, compte tenu de la nécessité de disposer d'un test de diagnostic des ESST particulièrement fiable, le Demandeur a poursuivi ses travaux.

Il a notamment établi que pour l'obtention d'un test de détection :
(i) sensible, c'est-à-dire ayant la capacité à identifier correctement les animaux non-infectés,
(ii) spécifique, c'est-à-dire ayant la capacité à identifier correctement les animaux infectés présentant des symptômes cliniques,
(iii) présentant une limite de détection la plus basse possible, c'est-à-dire apte à permettre la détection de petites quantités de PrPres, (détection de la PrPres avant l'apparition des symptômes cliniques) et
(iv) reproductible,
le traitement de l'échantillon biologique à analyser doit être réalisé dans des conditions qui permettent de conserver tout ou partie des motifs octa-peptides exclusivement dans la PrPres.

Il a en particulier trouvé, que pour effectivement répondre aux quatre conditions (i)-(iv) énoncées ci-dessus, il y a lieu de définir des conditions de traitement de l'échantillon à analyser précises qui éliminent totalement la PrPsens de l'échantillon, tout en permettant une capture spécifique de la PrPres, dans les conditions définies.

La présente invention a pour objet un procédé de diagnostic (Procédé A) d'une ESST ou maladie à prions provoquée par une souche d'ATNC par détection de la PrPres dans un échantillon biologique, caractérisé en ce qu'il comprend :
(1) le traitement dudit échantillon avec au moins une protéinase K, soit à une concentration comprise entre 30 µg/ml et 200 µg/ml pendant 10 minutes à 37°C pour un homogénat à 10 %, soit pendant une période et à une concentration équivalentes à la concentration comprise entre 30 µg/ml et 200 µg/ml dans les conditions précitées et de manière encore plus préférée pendant 10 minutes à une concentration comprise entre 30 µg/ml et 200 µg/ml pour un homogénat à 10 % ou pendant 30 minutes à une concentration comprise entre 10 µg/ml et 70 µg/ml, pour un homogénat à 10 %, ladite protéinase K étant mise en solution dans un tampon sélectionné dans le groupe constitué par les tampons d'homogénéisation de l'échantillon biologique et des tampons comprenant au moins l'un des agents suivants : au moins un agent tensioactif et/ou au moins un agent chaotrope et/ou au moins un sel, de manière à dégrader complètement la PrPsens, tout en conservant tout ou partie des motifs octa-peptide répétés de la PrPres, quelle que soit la souche d'ATNC,
(2) la mise en contact dudit échantillon traité avec un ligand desdits motifs octa-peptide et
(3) la détection de la présence éventuelle du complexe motifs octa-peptide répétés-ligand.

Il y a lieu de noter qu'un certain nombre de paramètres sont étroitement liés entre eux : la concentration en protéinase K dépend directement de la durée de traitement (temps d'incubation) de l'échantillon : l'on peut considérer qu'à 37°C par exemple, une incubation de 10 minutes avec une PK à une concentration comprise entre 30 et 200 µg/ml d'homogénat à 10% est équivalente à une incubation de 30 minutes avec une PK à une concentration comprise entre 10 µg/ml et 70 µg/ml d'homogénat à 10 %. Par exemple, une incubation pendant 30 minutes avec une PK à 25 µg/ml est équivalente à une incubation de 10 minutes avec une PK à une concentration de 75 µg/ml.

Quelle que soit la concentration en PK et la durée d'incubation, l'échantillon traité ne doit plus contenir de PrPsens non dégradée, alors que la PrPres, éventuellement présente, a conservé tout ou partie des motifs octa-peptides.

D'autres paramètres peuvent intervenir à un moindre degré (c'est-à-dire de manière non-essentielle), dans l'établissement de la concentration active de PK : il s'agit notamment du tampon dans lequel ladite PK est mise en solution ; lorsque la PK est mise en solution dans le tampon d'homogénéisation de l'échantillon, selon le tampon d'homogénéisation utilisé, la concentration minimale en PK pourra varier, dans la fourchette de concentrations précisées ci-dessus :
- dans un tampon glucosé ou dans de la guanidine (ou l'un de ses sels) la concentration minimale en PK est de préférence de 25 µg/ml (incubation 30 minutes) ou de 75 µg/ml (incubation 10 minutes)
- dans du tampon PBS, la concentration minimale en PK est de préférence de 50 µg/ml (incubation 30 minutes) ou de 150 µg/ml (incubation 10 minutes).

De manière avantageuse, la PK peut être mise en oeuvre dans un tampon différent du tampon d'homogénéisation ; un tel tampon comprend avantageusement au moins un agent tensioactif et/ou au moins un agent chaotrope et/ou au moins un sel.

Également de manière avantageuse, après le traitement (ou l'incubation) de l'échantillon avec la protéinase K, la suspension obtenue est avantageusement traitée dans les conditions définies dans la Demande internationale 99/41280, à savoir:
- addition à ladite suspension d'un tampon B, tel que défini dans cette demande internationale 99/41280 et notamment les alcools en C₃-C₆ et les mélanges d'alcool dont la constante diélectrique théorique moyenne est comprise entre 10 et 25,
- la centrifugation de la suspension obtenue et
- la solubilisation du culot dans un tampon comprenant au moins un agent tensioactif et/ou au moins un agent chaotrope, dans les conditions définies dans ladite Demande internationale 99/41280.

Un tel test a l'avantage d'être particulièrement adapté au diagnostic différentiel des ESST chez un même hôte, provoquées par différentes souches d'ATNC, et notamment au diagnostic différentiel des souches d'ESB par rapport aux souches habituelles de tremblante chez le mouton.

Il a, par exemple été montré que la souche ESB a infecté des humains en Grande-Bretagne par l'analyse des profils électrophorétiques de la PrPres, qui a montré des différences de migration en fonction des souches d'ATNC ; un profil caractéristique (type 4) a été retrouvé chez les patients développant une nouvelle variante de la maladie de Creutzfeldt-Jakob (vMCJ) qui semble liée au passage dans l'alimentation humaine de bovins infectés par l'agent de l'ESB (Collinge et al., Nature, 1996). Un profil similaire a été observé chez un macaque infecté par l'agent de l'ESB (Lasmezas et al., 1996) et un chat vraisemblablement contaminé par cet agent (Priola et al., Nature Med., 1996).

Toutefois, il s'agit d'une méthode longue et délicate à mettre en oeuvre si l'on veut obtenir des résultats reproductibles, ce qui constitue vraisemblablement un élément de la controverse qui existe concernant la classification des différents types de PrPres et l'utilisation de cette méthode (Parchi et al., Nature, 1997).

Il existe également une forte suspicion de contamination des moutons par l'agent de l'ESB (Butler, Nature, 1998). Ces animaux sont en effet sensibles à cet agent, alors qu'il existe chez les ovins une maladie endémique, la tremblante (scrapie des anglo-saxons), une autre ESST, qui présente des caractéristiques cliniques et histologiques proches et non différentiables. La seule méthode de référence pour un diagnostic différentiel entre l'agent de l'ESB et de la tremblante, est l'inoculation à la souris et l'étude du profil lésionnel, ce qui nécessite environ deux ans d'attente et qui n'a été réalisé en Grande-Bretagne que sur 9 moutons pour une population de plusieurs dizaines de millions de têtes.

De premiers essais ont été effectués pour essayer de distinguer les différentes souches d'ATNC dans un échantillon ; Kuczius T. et al., 1999 ont considéré que les variations observées entre les différentes souches d'ESST (scrapie, ESB et CJD) par la technique du glycotypage ne permettent pas de distinguer chaque souche ; ils ont sélectionné d'autres marqueurs biochimiques et biologiques de la PrPres qui permettent de mieux distinguer entre elles les différentes souches de prions. Les paramètres d'analyse qu'ils ont utilisés sont les suivants : résistance à long terme à la protéinase K, masse moléculaire de la PrPres, topologie et quantité des dépôts de PrPres. Les résultats obtenus montrent que les PrPres de différentes souches d'ESB et de scrapie présentent des différences significatives dans leur résistance à long terme à la protéinase K. Selon la souche de scrapie, la résistance à la PK varie : faible résistance : souche Chandler ; résistance intermédiaire : souche 22A ; relative stabilité : souche 87V. Dans les mêmes conditions, les souches d'ESB présentent une résistance intermédiaire. Alors que le glycotypage ne permet pas de distinguer entre la souche de scrapie 87V et les souches d'ESB, ces deux types de souches sont clairement distinguables après traitement à long terme à la PK. Toutefois, il ne s'agit pas de protocoles suffisamment fiables et utilisables à grande échelle et sur le terrain.

Dans ce contexte, il est important de disposer d'une méthode de détection des PrPres fiable, sensible, permettant un diagnostic différentiel, peu coûteuse et facile à mettre en oeuvre, à partir d'un échantillon biologique, tel qu'un échantillon tissulaire.

La présente invention a, en conséquence, également pour objet un procédé de diagnostic différentiel (procédé B) des ESST provoquées par des souches d'ATNC, dans un échantillon biologique, par détection des PrPres associées aux différentes souches d'ATNC, caractérisé en ce qu'il comprend :
(a) la détection de la PrPres d'une souche d'ATNC dans une première fraction dudit échantillon, conformément aux étapes (1) à (3) suivantes :
   (1) le traitement dudit échantillon avec au moins une protéinase K, de manière à dégrader complètement la PrPsens, tout en conservant tout ou partie des motifs octa-peptide répétés de la PrPres, quelle que soit la souche d'ATNC,
   (2) la mise en contact dudit échantillon traité avec un ligand desdits motifs octa-peptide et
   (3) la détection de la présence éventuelle du complexe motifs octa-peptide répétés-ligand, puis :
(b) pour chaque échantillon pour lequel la présence d'un complexe motifs octa-peptide répétés-ligand est détectée à l'étape (a) :
   - le traitement d'une deuxième fraction dudit échantillon avec au moins une protéinase K, de manière à ce que la majorité des motifs octa-peptide répétés soit éliminée pour la PrPres associée à au moins une souche d'intérêt, notamment la souche de l'ESB et où l'ensemble des PrPres associées aux autres souches d'ATNC conservent tout ou partie desdits motifs octa-peptide répétés,
   - la mise en contact de ladite deuxième fraction dudit échantillon traité, avec un ligand desdits motifs octa-peptide répétés et
   - la détection de la présence éventuelle du complexe motifs octa-peptide répétés-ligand.

La présente invention a en outre pour objet un procédé de diagnostic différentiel (variante du procédé B) des ESST provoquées par des souches d'ATNC dans un échantillon biologique considéré comme contenant de la PrPres (test de détection effectué par n'importe quelle méthode), caractérisé en ce qu'il comprend pour chaque échantillon pour lequel la présence d'une PrPres a été détectée, la mise en oeuvre de l'étape (b) telle que définie ci-dessus,
- la mise en contact de ladite deuxième fraction dudit échantillon traité, avec un ligand desdits motifs octa-peptide répétés, et
- la détection de la présence éventuelle du complexe motifs octa-peptide répétés-ligand.

Selon un mode de mise en oeuvre avantageux dudit procédé, le traitement avec ladite protéinase K selon l'étape (a) ou (b) est réalisé pendant 30 secondes à 2 heures, à une température inférieure à 80°C, de préférence entre 10 minutes et 30 minutes.

Selon un disposition avantageuse de ce mode de mise en oeuvre, le traitement avec ladite protéinase K est réalisé soit à une concentration comprise entre 30 µg/ml et 200 µg/ml pendant 10 minutes à 37°C pour un homogénat à 10 %, soit pendant une période et à une concentration équivalentes à la concentration comprise entre 30 µg/ml et 200 µg/ml dans les conditions précitées et de manière encore plus préférée pendant 10 minutes à une concentration comprise entre 30 µg/ml et 200 µg/ml pour un homogénat à 10 % ou pendant 30 minutes à une concentration comprise entre 10 µg/ml et 70 µg/ml, pour un homogénat à 10 %.

Selon un autre mode de mise en oeuvre avantageux dudit procédé, la protéinase K est mise en solution dans un tampon sélectionné dans le groupe constitué par les tampons d'homogénéisation de l'échantillon biologique et des tampons comprenant au moins l'un des agents suivants : au moins un agent tensioactif et/ou au moins un agent chaotrope et/ou au moins un sel.

Dans le cadre du diagnostic différentiel (procédé B), outre la concentration en PK, d'autres conditions peuvent éventuellement intervenir sur la dégradation des motifs octa-peptides, en fonction de la souche : en effet, lorsque la PK est mise en oeuvre dans un tampon différent du tampon d'homogénéisation, tel que dans un tampon qui comprend avantageusement au moins un agent tensioactif et/ou au moins un agent chaotrope et/ou au moins un sel, le nombre de motifs octa-peptides dégradés peut varier en fonction de la composition dudit tampon et de la concentration des différents agents.

On entend par souche d'intérêt, la ou les souches d'ATNC, la souche d'ESB notamment, pour lesquelles l'on souhaite éliminer les motifs octa-peptide répétés, par exemple.

Une des caractéristiques principales des procédés A et B est de jouer sur les conditions dans lesquelles est effectué le traitement protéasique de façon à contrôler la dégradation des motifs octapeptides répétés. Selon l'usage envisagé, on fera en sorte que ces motifs soient conservés (procédé A) ou détruits (procédé B) :
- dans le cadre du procédé A, ce contrôle visera à ce que tout ou partie des motifs octapeptide répétés soient conservés dans le but de favoriser une détection très sensible de la PrPres.
- dans le cadre du procédé B, le contrôle de la dégradation par la protéase visera à ce que la majorité et éventuellement la totalité des motifs octa-peptide répétés soit dégradée pour la PrPres correspondant à la/les souche(s) d'intérêt, quelle que soit l'espèce dans laquelle elle est exprimée, dans des conditions ou tout ou partie des motifs octa-peptide répétés sont conservés pour les PrPres correspondant à toutes les autres souches d'ESST. Dans ce cas, l'intérêt de l'invention est de permettre un diagnostic différentiel de la souche d'intérêt par rapport aux autres souches d'ATNC.

En effet :

De manière surprenante, dans les conditions selon l'étape (1) ou l'étape (a), la PK n'entraîne pas le clivage de tout ou partie des motifs octa-peptide répétés des PrPres associées à toutes les souches d'ATNC ou prions, alors que les conditions de l'étape (b) entraînent le clivage des motifs octa-peptide répétés de la PrPres associée à la souche d'intérêt, alors que l'ensemble des PrPres associées aux autres souches d'ATNC conservent tout ou partie desdits motifs octa-peptides répétés.

Également de manière surprenante, un tel test, notamment lorsque le ligand est un anticorps, présente les avantages suivants :
- une grande sensibilité de détection car les anticorps dirigés contre ces motifs octa-peptide répétés ont une beaucoup plus grande affinité que les anticorps dirigés contre d'autres régions de la PrPres 27-30 et sont plus résistants aux tampons utilisés ; en effet, la reconnaissance d'un motif répété favorise des interactions de haute affinité et offre la possibilité de fixer plusieurs molécules d'anticorps sur une seule molécule de PrP ;
- la possibilité d'un diagnostic différentiel des souches d'ATNC ou prions car il est possible selon les conditions mises en oeuvre d'obtenir ou non la digestion de ces motifs ; il est notamment possible de les éliminer pour toutes les maladies liées à la souche d'intérêt, l'agent de l'ESB par exemple, alors qu'il est possible de la conserver pour les autres maladies dites à prions. De ce fait, les méthodes selon l'invention proposent un test simple de diagnostic différentiel de l'ESB et/ou d'une autre souche d'intérêt par rapport aux autres souches en mettant en oeuvre deux conditions différentes (étape (1) ou (a) et étape (b)), les conditions de l'étape (1) ou (a) (conservant les motifs octa-peptide répétés des PrPres associées à l'ensemble de souches d'ATNC) permettant la détection de toutes les souches et les conditions de l'étape (b) (éliminant ces motifs uniquement dans la PrPres associée à une souche d'intérêt) ne révélant que les autres souches.

De tels tests trouvent donc notamment application dans la recherche de la contamination du mouton par la souche d'ESB que l'on ne sait pas habituellement distinguer des souches classiques de tremblante (scrapie).

Selon un mode de mise en oeuvre avantageux desdits procédés, la PK est mise en solution dans le tampon qui comprend de préférence :
**a. au moins un agent tensioactif,** sélectionné dans le groupe constitué par :
   - des tensioactifs anioniques, tels que le SDS (dodécylsulfate de sodium), le sarkosyl (lauroyl-sarcosine), le cholate de sodium, le désoxycholate de sodium, le taurocholate de sodium ;
   - des tensioactifs zwitterioniques, tels que le SB 3-10 (décyl-sulfobétaïne), le SB 3-12 (dodécyl-sulfobétaïne), le SB 3-14, le SB 3-16 (hexadécyl-sulfobétaïne), le CHAPS et le désoxyCHAPS ;
   - des tensioactifs non-ioniques, tels que le C12E8 (dodécyl-octa-éthylèneglycol), le Triton^{®} X100, le Triton^{®} X114, le Tween^{®} 20, le Tween^{®} 80, le MEGA 9 (nonanoyl-méthylglucamine), l'octylglucoside, le LDAO (dodécyl-diméthylamine oxyde) ou le NP40 ou
   - des mélanges de tensioactifs tels qu'un mélange d'un agent tensioactif ionique et d'un agent tensioactif non-ionique, un mélange de deux agents tensioactifs ioniques ou un mélange d'un agent tensioactif ionique et d'un agent tensioactif zwitterionique et/ou
**b. au moins un agent chaotrope** sélectionné dans le groupe constitué par l'urée et la guanidine ou un mélange de ceux-ci et/ou
c. au moins un sel sélectionné parmi les sels de métaux alcalins ou non.

Selon une disposition avantageuse de ce mode de mise en oeuvre, ledit tampon comprend au moins 5% de tensioactif anionique, de préférence du sarkosyl, éventuellement associé à du SDS.

Selon un autre mode de mise en oeuvre avantageux desdits procédés, le ligand est sélectionné dans le groupe constitué par des aptamères et des anticorps qui se lient à la région des motifs octapeptide répétés.

Selon un autre mode de mise en oeuvre avantageux desdits procédés, le traitement de l'étape (b) est réalisé dans les mêmes conditions que celles définies à l'étape (a) (1), mais à une concentration en PK supérieure à celle utilisée à l'étape (a) (1).

La présente invention a, en outre, pour objet une trousse de diagnostic pour la mise en oeuvre des procédés selon l'invention, caractérisée en ce qu'elle comprend en association au moins un agent tensioactif et/ou au moins un agent chaotrope et /ou au moins un sel et une protéase tels que définis ci-dessus.

Le complexe motifs octa-peptides répétés-anticorps (dans le cas où le ligand est un anticorps) est détecté par des méthodes immunologiques standards.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 représente les différentes séquences de PrP : humaine, ovine, bovine, murine et cricétidienne ;
- la figure 2 représente la détection de la PrPres par Western blot ;
- les figures 3 à 6 correspondent à différentes conditions selon l'étape (1) ou (a) d'une part et l'étape (b) d'autre part, chez l'homme (figures 3 et 4) et chez les ruminants (figures 5 et 6), dans le cas où la présence des motifs octa-peptide répétés est détectée par un dosage immunométrique à deux sites.
- les figures 7-12 illustrent l'influence des tampons dans la détection différentielle de l'ESB et de la tremblante.
- les figures 13 et 14 illustrent l'influence de la composition des tampons et de la concentration en protéinase K (PK) pour la détection des différents type de MCJ.
- la figure 15 illustre les résultats obtenus par une digestion directe d'homogénats de cerveau par la protéinase K.
- la figure 16 illustre les différences de résistance de la PrPres à la protéinase K en fonction des types de MCJ.
- la figure 17 illustre la détection par Western-blot de PrPres digérée par de la PK et purifiée sous forme de SAF.

Il doit être bien entendu, toutefois, que ces exemples sont donnés uniquement à titre d'illustration de l'objet de l'invention, dont ils ne constituent en aucune manière une limitation.

### EXEMPLE 1 : Obtention et caractérisation des anticorps monoclonaux spécifiques du motif octapeptide répétitif.

### - Synthèse et marquage du peptide.

Un peptide représentatif du motif octa-peptide répété de la PrP, par exemple le motif G-G-W-G-Q-P-H-G-G-G-W-G-Q-G-_{(NH2)}, correspondant à la séquence 79-92 de la PrP humaine, a été synthétisé à l'aide d'un synthétiseur automatique (Milligen 9050, Waters, Milford, MI). Le peptide a été couplé de façon covalente avec l'acétylcholinestérase (AchE) par l'intermédiaire d'un réactif hétérobifonctionnel, le succinimidyl 4-(*N*-maleimidomethyl) cyclohexane-1-carboxylate (SMCC, Calbiochem, France), comme décrit précédemment pour d'autres peptides ou protéines (McLaughlin et al., 1987, Grassi et al., 1989). Cette méthode implique la réaction d'un groupement thiol introduit dans le peptide avec la fonction maléimide qui a été fixée sur l'AchE par réaction avec le SMCC. Le groupement thiol a été introduit dans le peptide par réaction avec le N-succinimidyl S-acétylthioacétate (SATA) comme décrit précédemment (McLaughlin et al., 1987). Le couplage a été obtenu en faisant réagir l'AChE-SMCC avec un excès de peptide thiolé.

### - Immunisation et préparation des anticorps monoclonaux

Une préparation de *"Scrapie-associated fibrils"* (SAFs, préparation de PrPres) a été obtenue à partir de cerveaux de hamster infectés (souche de tremblante 263K) comme décrit précédemment (Lasmezas et al., 1997). Cette préparation a été inactivée par traitement à l'acide formique avant immunisation des souris. Des souris invalidées (*knock-out*) pour le gène de la PrP (souris PrP^{0/0}) ont été immunisées avec ces préparations de SAF et des cellules d'hybridome ont été préparées comme décrit précédemment (Grassi et al., 1988, 1989). Le criblage des surnageants de culture a été réalisé comme décrit ci-dessus. 57 hybridomes ont pu être identifiés et stabilisés ; ils ont été appelés SAF-1 à SAF-90. Tous ces anticorps se sont avérés reconnaître les SAFs immobilisés sur les plaques de microtitration alors qu'une minorité d'entre eux ont démontré une capacité à reconnaître des conjugués peptide-AchE. Parmi ces derniers, sept reconnaissent clairement le motif octa-peptide répété (peptide 79-92) ; il s'agit des anticorps SAF-15, SAF-31, SAF-32, SAF-33, SAF-34, SAF-35, SAF-37. La liste des anticorps obtenus ainsi que leurs principales caractéristiques sont présentées dans le Tableau ci-après. Après clonage et expansion sous la forme de liquide d'ascite, les anticorps monoclonaux ont été purifiés par chromatographie d'affinité sur colonne de Protéine A-SEPHAROSE^{®} et conservés à -20°C jusqu'à utilisation. L'isotype des anticorps a été déterminé par immunodiffusion radiale selon la technique d'Ouchterlony.

### - Criblage des surnageants de culture d'hybridome

La présence d'anticorps spécifique de la PrP dans les surnageants de culture d'hybridome a été mise en évidence de deux façons, en testant soit leur capacité à lier des conjugués peptide-AchE soit des SAFs de hamster. Dans le premier cas, le criblage a été réalisé dans des plaques contenant un anticorps anti-IgG de souris immobilisé comme décrit précédemment (Créminon et al., 1993, Frobert et al., 1991). En résumé, 100 µl de surnageants de culture et 100 µl de conjugué Peptide-AchE ont été mis à réagir une nuit à +4°C dans des plaques contenant des anticorps de chèvre anti-IgG de souris immobilisés. Après lavage des plaques, 200 µl de réactif d'Ellman (Ellman et al. 1961) ont été rajoutés dans les puits afin de détecter la présence d'AchE fixée sur la phase solide. Dans le deuxième cas, des plaques contenant une préparation de SAF immobilisée ont été préparées en faisant réagir 50 µl d'une solution à 2 µg/ml dans un tampon phosphate 0,05 M, pH 7,4 pendant une nuit à température ambiante. Après lavage, les plaques ont été saturées avec le tampon EIA (tampon phosphate 100 mM, pH 7,4 contenant du NaCl 150 mM, 0,1% de la sérum albumine bovine (BSA) et de l'azoture de sodium 0,01%) pendant une nuit à +4°C et ont été conservées à cette température jusqu'à leur utilisation. La liaison des anticorps monoclonaux sur les SAFs immobilisés a été mise en évidence à l'aide d'anticorps de chèvre anti-IgG de souris marqués à l'AchE comme décrit précédemment (Negroni et al., 1998).

**Tableau: Anticorps monoclonaux produits contre une préparation de SAF de hamster.**

| **AcMs** | **Isotype** | **Peptide Reconnu** | **AcMs** | **Isotype** | **Peptide reconnu** |
|---|---|---|---|---|---|
| **SAF 1** | IgM | X | **SAF 54*** | IgG2b | 142-160 |
| **SAF 2** | ? | A | **SAF 56** | IgM | A |
| **SAF 3** | IgG2a | X | **SAF 58** | IgM | A |
| **SAF 4** | IgG2a | A | **SAF 59** | IgM | A |
| **SAF 5** | IgG1 | X | **SAF 60*** | IgG2b | 142-160 |
| **SAF 7** | IgG2a | X | **SAF 61** | IgG2a | 142-160 |
| **SAF 8** | IgG1 | A | **SAF 63** | IgM | A |
| **SAF 9** | IgG1 | A | **SAF 65** | IgG1(?) | A |
| **SAF 10** | IgG1 | A | **SAF66** | IgG2a | 142-160 |
| **SAF 11** | IgG2a | X | **SAF 67** | IgG1 | X |
| **SAF 12** | IgM | A | **SAF 68** | IgG2a | A |
| **SAF 13** | IgM | A | **SAF 69*** | IgG2b | 142-160 |
| **SAF 14** | IgG1 | A | **SAF 70*** | IgG2b | 142-160 |
| **SAF 15*** | IgG3 | 79-92 | **SAF 72** | IgG2b | A |
| **SAF 21** | IgG1 | X | **SAF 73** | IgG1 | X |
| **SAF 22** | ? | A | **SAF 75** | IgG2a | 142-160 |
| **SAF 23** | IgG1 | X | **SAF 76** | IgG2a | 142-160 |
| **SAF 24** | IgG1 | A | **SAF 77** | IeG1 | X |
| **SAF 31*** | IgG2b | 79-92 | **SAF 80** | IgG1 | X |
| **SAF 32*** | IgG2b | 79-92 | **SAF 81** | IgM | A |
| **SAF 33*** | IgG2b | 79-92 | **SAF 82** | IgG 1 | A |
| **SAF 34*** | IgG2a | 79-92 | **SAF 83*** | IgG1 | A |
| **SAF 35*** | IgG2b | 79-92 | **SAF84*** | IgG2b | A |
| **SAF 37*** | IgG2b | 79-92 | **SAF 85** | IgM | A |
| **SAF 42** | IgG1 | A | **SAF 91** | IgM | A |
| **SAF 44** | IgM | A | **SAF 94** | IgM | A |
| **SAF 50** | IgM | A | **SAF 95** | IgG1 | A |
| **SAF 51** | IgM | A | **SAF 96** | IgM | A |
| **SAF 53*** | IgG2a | X | | | |

| | | | | | |
|---|---|---|---|---|---|
| **79-92:** anticorps reconnaissant le peptide 79-92 **142-160:** anticorps reconnaissant le peptide 142-160 **Epitope X :** anticorps qui ne reconnaissent que les SAFs immobilisés **Epitope A :** anticorps qui reconnaissent le peptide 126-164 mais ne lient pas le peptide 142-160. * anticorps monoclonaux qui ont démontré leur capacité à reconnaître la PrPsens d'au moins une espèce testée pendant cette étude (humaine, bovine, ovine, souris et hamster) dans le cadre d'un dosage immunométrique. | | | | | |

### EXEMPLE 2 : Détection de la PrPres par Western blot

### I. Traitement de l'échantillon

(i) Préparation d'un homogénat de tissu, à partir des différents échantillons biologiques
   - vache ESB, mouton scrapie, homme vMCJ (type 4), homme MCJ sporadique (type 1) et contrôles négatifs correspondants
      On prélève 350 mg de cerveau bovin : on le broie et on l'homogénéise à 20 % (p/v) dans une solution de glucose à 5 %.
      Pour réaliser l'homogénéisation, le prélèvement de cerveau (350 mg) et 1,4 ml de solution de glucose sont introduits dans des tubes comprenant des billes de céramique, et agités fortement (Ribolyser Hybaid).
      On dilue les échantillons positifs dans un homogénat provenant de cerveaux sains de l'espèce correspondante comme suit :

   - pour le mouton : au 1/100^{ème}
   - pour la vache : au 1/50^{ème}
   - pour l'homme : type 1 ou 4 : au 1/40^{ème}; type 3 : au 1/80^{ème} ; type 2 au 1/20^{ème}
(ii) conditions de l'étape (1)
   On incube une première fraction (500 µl) d'homogénat à 20 % obtenu en (i) avec 500 µl d'un tampon comprenant du sarkosyl à 10 % (SK10), du Triton^{®} X100 à 10 % (T10), de l'urée 2M (U2) et de la protéinase K (PK) à 60 µg/ml de tampon (PK1) pendant 10 minutes à 37°C (correspondant à 30 µg/ml final pour un homogénat à 10 %).

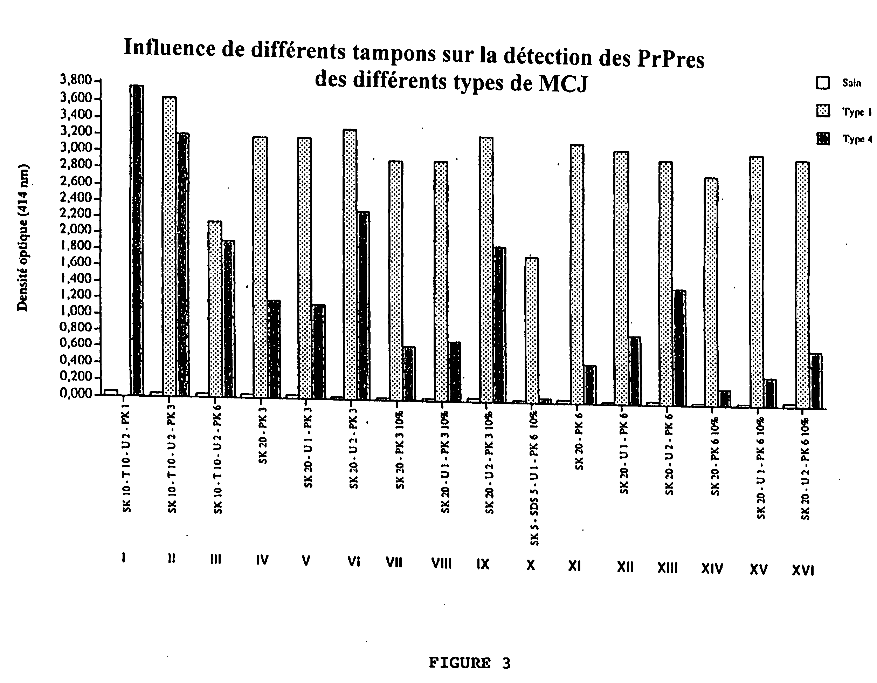

   Dans les figures 3-6, PK3 correspond à une concentration en PK de 180 µg/ml de tampon et PK6 correspond à une concentration en PK de 360 µg/ml de tampon.
(iii) on ajoute 500 µl d'un tampon constitué de butanol-1 (correspondant aux tampons B, tels que décrits dans la Demande internationale WO 99/41280) ; puis on centrifuge à 15000 rpm pendant 5 min. (environ 17 000 g).
(iv) le culot de centrifugation qui contient la PrPres est repris dans 80-100 µl d'un tampon C tel que décrit dans la Demande internationale WO 99/41280, de préférence un tampon de Laemmli contenant 4 % de SDS et chauffé à 100°C pendant 5 min., pour réaliser un Western blot ou repris, pour effectuer un dosage immunométrique, successivement dans un tampon C1 comprenant de l'urée 6M et du sarkosyl 0,5 %, suivi d'un chauffage pendant 5 min. à 100°C, puis dans un tampon C2 comprenant de la guanidine 2M, suivi d'un chauffage pendant 5 min. à 100°C.
(v) conditions de l'étape (b)
   On incube une deuxième fraction (500 µl) d'homogénat à 20 % obtenu en (i) avec 500 µl d'un tampon comprenant du sarkosyl à 5 % (SK5), du SDS à 5 % (SDS5), de l'urée 1M (U1) et de la protéinase K (PK) à 360 µg/ml (PK6) pendant 10 min. à 37°C, puis on réalise les étapes (iii) et (iv) ci-dessus.

### II. Western blot

Les échantillons obtenus sont utilisés pour réaliser une électrophorèse SDS-PAGE et transférés sur membrane de nitrocellulose dans les conditions exposées à l'exemple 1 et à l'exemple 3 de la Demande internationale WO 99/41280 précitée.

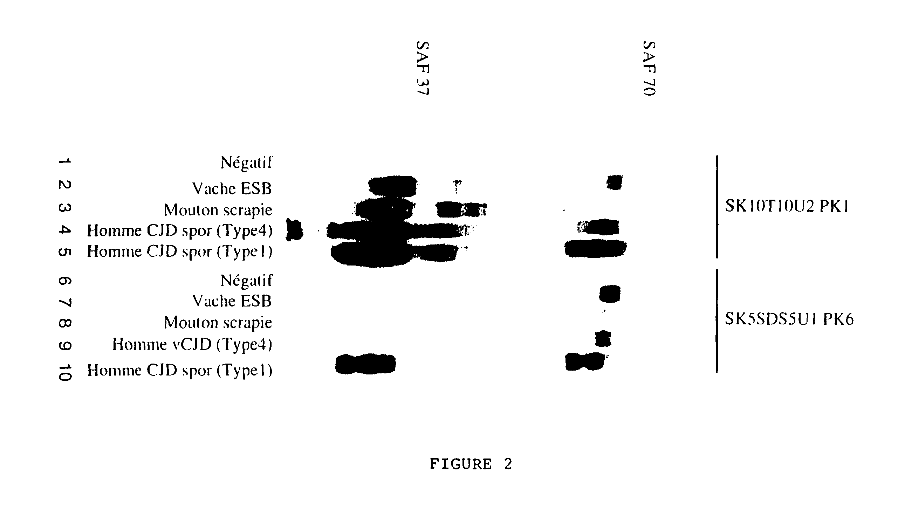

L'immunodétection de la PrPres est réalisé avec les anticorps monoclonaux SAF70 et SAF37 tels que décrits à l'exempte 1, ci-dessus et des Ig de chèvre anti-lapin conjuguées à de la peroxydase (1/2500). L'immunoréactivité est révélée par chimioluminescence (ECL. Amlersham), quantifiée et visualisée sur films autoradiographiques, comme illustré sur la figure 2.

A cette figure :
* les pistes 1-5 correspondent à des conditions selon l'étape (1) ou (a) (SK10+T10+U2+PK1) :
   - Piste 1 :: contrôle négatif
   - Piste 2 :: Vache ESB
   - Piste 3 :: mouton scrapie
   - Piste 4 :: homme vMCJ (type 4)
   - Piste 5 :: homme MCJ (type 1) et
* les pistes 6-10 correspondent à des conditions selon l'étape (b) (SK10+SDS5+U1+PK6) :
   - Piste 1 :: contrôle négatif
   - Piste 2 :: Vache ESB

   - Piste 3 :: mouton scrapie
   - Piste 4 :: homme vMCJ (type 4)
   - Piste 5 :: homme MCJ (type 1) et

Les résultats obtenus montrent que :
- à l'étape (1) ou (a) (pistes 1 à 5), la PrPsens est systématiquement détruite, alors que le signal obtenu avec la PrPres est systématiquement supérieur, avec l'anticorps dirigé contre les motifs octa-peptide répétés, au signal obtenu sur les mêmes échantillons avec un anticorps dirigé contre la région 142-160 de la PrP ;
- à l'étape (b) (pistes 6 à 10), la PrPsens est systématiquement détruite, alors que le signal obtenu aux pistes 8 et 10 (en présence de l'anticorps dirigé contre les motifs octa-peptide répétés) est similaire ou supérieur au signal obtenu sur les mêmes échantillons avec un anticorps dirigé contre la région 142-160 de la PrP, tandis qu'il est inférieur et même indétectable sur les pistes 7 et 9 (PrPres de l'ESB).

### EXEMPLE 3 : Détection de la PrPres avec un dosage immunométrique à deux sites utilisant comme anticorps de capture un anticorps monoclonal reconnaissant les motifs octa-peptide répétés.

Pour réaliser un dosage immunométrique à deux sites, le culot obtenu en (iv) à l'exemple 2 est par exemple dissous dans un tampon comprenant du sarkosyl (0,25-1 %) et de l'urée (0,25-8 M) ou du SDS (0,25-1 %) et de l'urée (0,25-1 M) ; l'échantillon obtenu sera de préférence dilué (au ¼ ou au ½), après chauffage avec un tampon contenant de l'albumine conduisant à une concentration finale en albumine comprise entre 0,1 et 1 % (p/v) ou avec un tampon contenant du désoxycholate à 1 %).

Le dosage immunométrique à deux sites est réalisé dans des plaques de microtitration contenant un anticorps immobilisé dans les conditions déjà décrites pour d'autres protéines (Grassi et al., 1989). Son principe est le suivant : la PrP analysée est reconnue par un anticorps fixé sur le support solide (anticorps de capture) et par un deuxième anticorps, reconnaissant une autre partie de la molécule, qui est marqué avec une enzyme (ici l'acétylcholinestérase, anticorps traceur), à 5 unités Ellman/ml (1 unité Ellman = 7,35x10⁻² unités enzymatiques).

Dans le cadre de l'invention, l'anticorps de capture est dirigé contre les motifs octa-peptide répétés et l'anticorps traceur reconnaît une séquence comprise dans la région 94-230 de la PrP, par exemple la zone 142-160 de la PrP. Après lavage de la phase solide, l'activité enzymatique fixée sur la plaque est proportionnelle à la quantité de PrPres possédant les motifs octa-peptide répétés initialement dans l'échantillon analysé.

En pratique, le dosage est réalisé de la façon suivante :
100 µl de la solution de PrP à analyser sont déposés dans les puits de la plaque de microtitration contenant l'anticorps reconnaissant les motifs octa-peptide répétés. Après 3 heures de réaction à température ambiante, la plaque est lavée avant ajout de 100 µl d'une solution de l'anticorps traceur (5 unités Ellman/ml ; 1 unité Ellman = 7,35x10⁻² unités enzymatiques). Après une nuit de réaction à +4°C, les plaques sont lavées à nouveau avant ajout de 200 µl d'une solution de substrat (réactif d'Ellman, Grassi et al., 1989) qui va permettre de mesurer l'activité de l'acétylcholinestérase fixée sur la phase solide. Après 30 minutes de réaction enzymatique, l'absorbance (D.O. à 414 nm) de chaque puits est mesurée.

### EXEMPLE 4 : Étude comparative de différentes conditions : étape (1) ou (a) et étape (b).

Les échantillons sont préparés comme décrits à l'exemple 2.

Les homogénats sont préparés comme à l'exemple 2.

### - chez l'homme

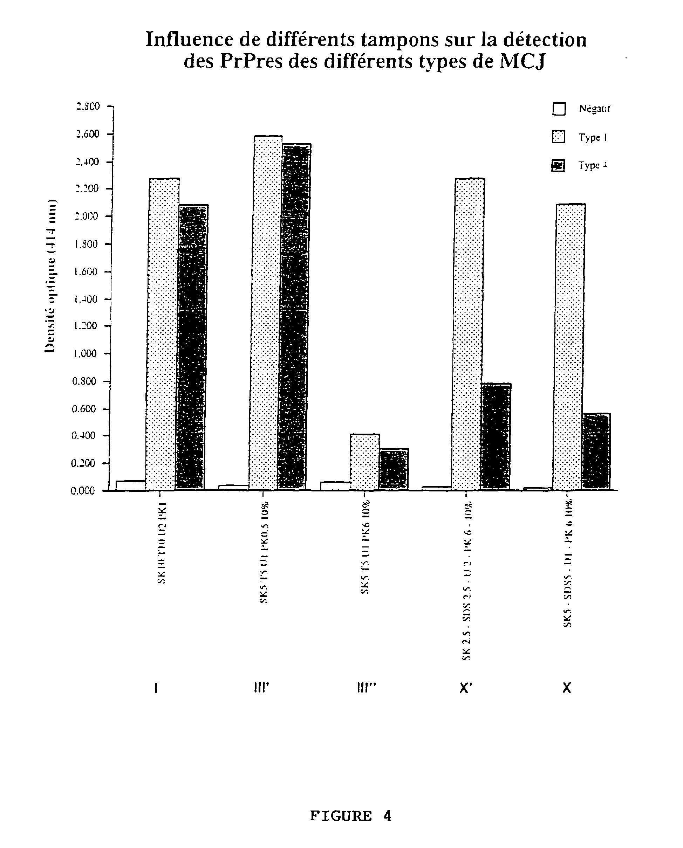

Les figures 3 et 4 illustrent les résultats obtenus avec différents tampons :
* conditions selon l'étape (1) ou (a) du procédé selon l'invention :
   I : homogénat à 20%+SK10+T10+U2+PK1
   III' : homogénat à 10%+SK5+T5+U1+PK0,5 (figure 4)
* conditions selon l'étape (b) du procédé selon l'invention :
   II : homogénat à 20%+SK10+T10+U2+PK3
   III : homogénat à 20%+SK10+T10+U2+PK6
   III'' : homogénat à 10%++SK5+T5+U1+PK6 (figure 4)
   IV : homogénat à 20%+SK20+PK3
   V : homogénat à 20%+SK20+U1+PK3
   VI : homogénat à 20%+SK20+U2+PK3
   VII : homogénat à 10%+SK20+PK3
   VIII : homogénat à 10%+SK20+U1+PK3
   IX : homogénat à 10%+SK20+U2+PK3
   X : homogénat à 10%+SK5+SDS5+U1+PK6
   X' : homogénat à 10%+SK2,5+SDS2,5+U2+PK6 (figure 4)
   XI : homogénat à 20%+SK20+PK6
   XII : homogénat à 20%+SK20+U1+PXC6
   XIII : homogénat à 20%+SK20+U2+PK6
   XIV : homogénat à 10%+SK20+PK6
   XV : homogénat à 10%+SK20+U1+PK6
   XVI : homogénat à 10%+SK20+U2+PK6

La quantité de PrPres ayant conservé le motif octa-peptide répété est mesurée à l'aide du dosage immunométrique à deux sites (absorbance ou D.O. à 414 nm, voir exemple 3 ; on mesure l'apparition d'une coloration jaune, réactif d'Eilman) : contrôle négatif (□), MCJ sporadique de type 1 (□) et vMCJ de type 4 (■)).

### - chez les ruminants

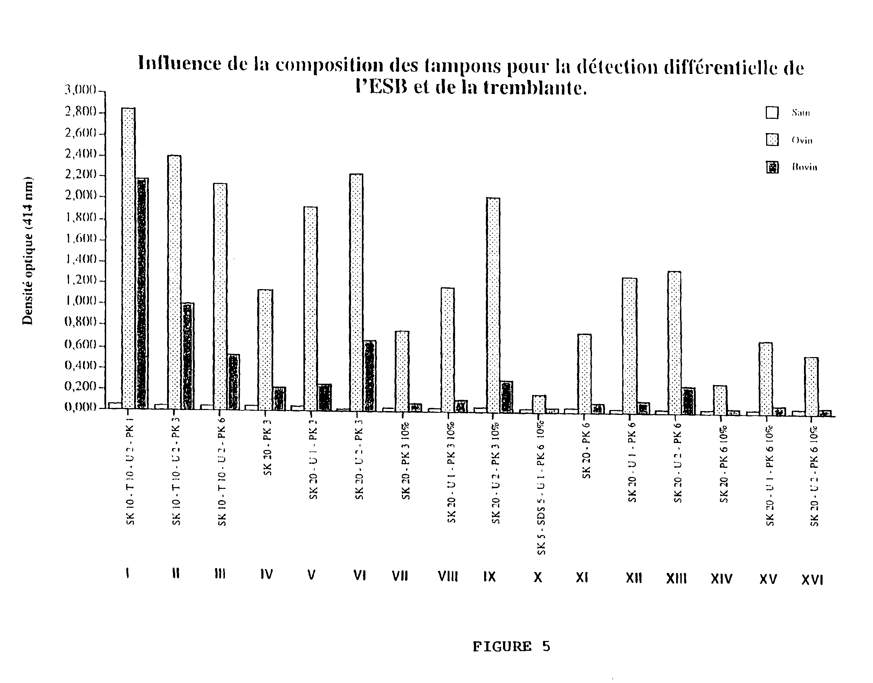

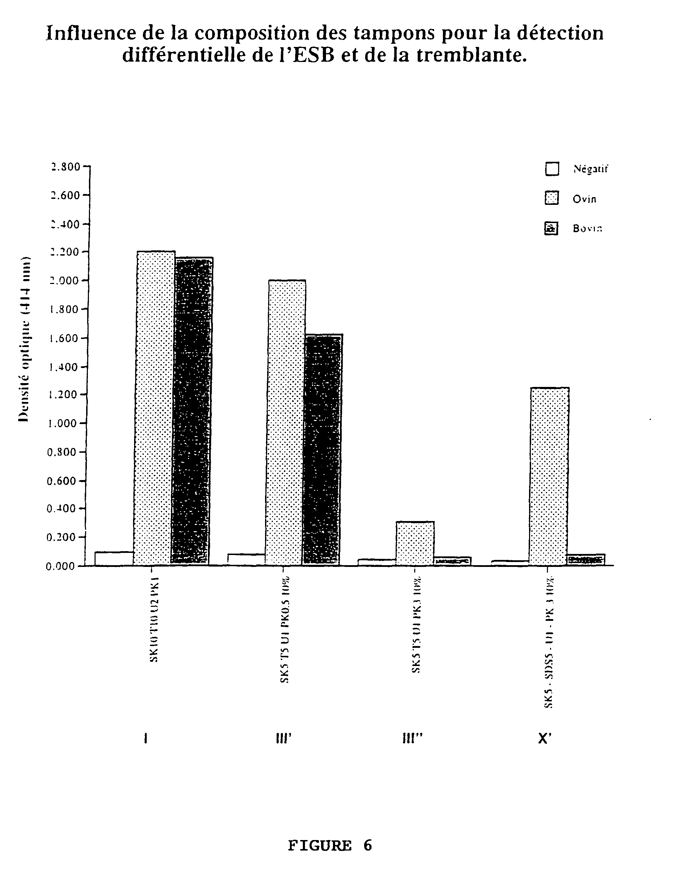

Les figures 5 et 6 illustrent les résultats obtenus avec différents tampons :
* conditions selon l'étape (1) du procédé selon l'invention :
   I : homogénat à 20%+SK10+T10+U2+PK1
   III' : homogénat à 10%+SK5+T5+U1+PK0,5 (figure 6)
* conditions selon l'étape (b) du procédé selon l'invention :
   II. homogénat à 20%+SK10+T10+U2+PK3
   III : homogénat à 20%+SK10+T10+U2+PK6
   III" : homogénat à 10%++SK5+T5+U1+PK3 (figure 6)
   IV : homogénat à 20%+SK20+PK3
   V : homogénat à 20%+SK20+U1+PK3
   VI : homogénat à 20%+SK20+U2+PK3
   VII : homogénat à 10%+SK20+PK3
   VIII : homogénat à 10%+SK20+U1+PK3
   IX : homogénat à 10%+SK20-U2+PK3
   X : homogénat à 10%+SK5+SDS5+U1+PK6
   X' : homogénat à 10%+SK5+SDS5+U1+PK3 (figure 5)
   XI : homogénat à 20%+SK20+PK6
   XII : homogénat à 20%+SK20+U1+PK6
   XIII : homogénat à 20%+SK20+U2+PK6
   XIV : homogénat à 10%+SK20+PK6
   XV : homogénat à 10%+SK20+U1+PK6
   XVI : homogénat à 10%+SK20+U2+PK6

La quantité de PrPres ayant conservé le motif octa-peptide répété est mesurée à l'aide du dosage immunométrique à deux sites (absorbance ou D.O. à 414 nm, voir exemple 3) : contrôle négatif (□), ATNC bovin ( 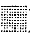 ) et ATNC ovin (■).

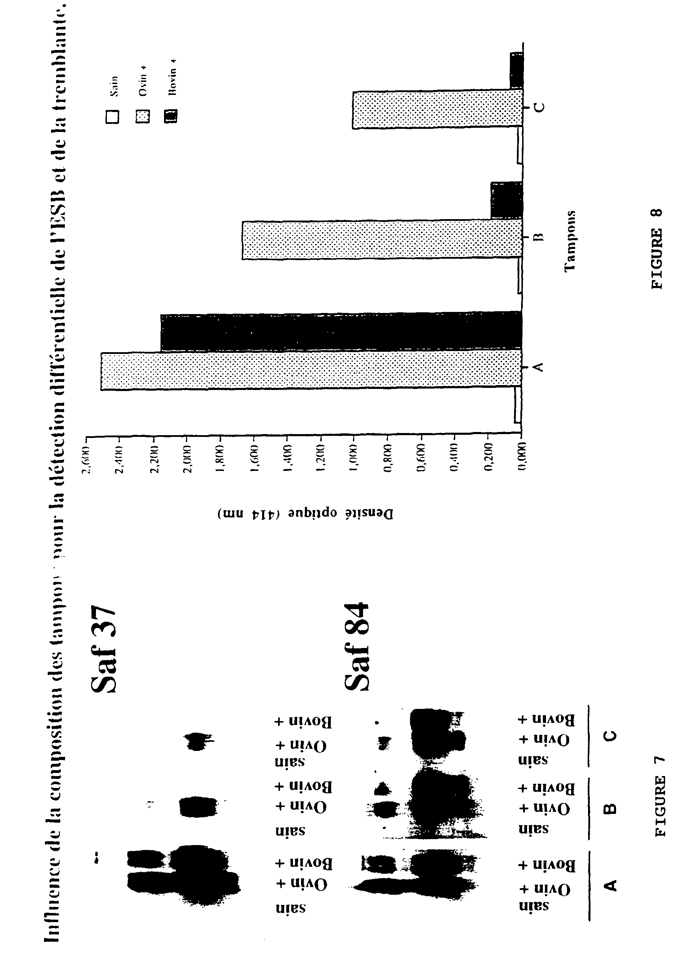

### - Figures 7 (Western blot) et 8 (dosage immunométrique à deux sites) :

- La comparaison est effectuée à partir d'homogénats à 20 % de cerveaux de mouton sain, de mouton atteint de tremblante et de bovin atteint d'ESB, obtenus, dans les conditions exposées à l'exemple 2.
- Traitement des échantillons :
   - **A :**: sarkosyl 10 %+Triton 10 %+urée 2M+protéinase K 60 µg/ml, 10 minutes
   - **B :**: sarkosyl 10 %+urée 2M+protéinase K 240 µg/ml, 10 minutes
   - **C :**: sarkosyl 10 %+ protéinase K 240 µg/ml, 10 minutes
- Détection
   par Western blot (figure 7) : anticorps Saf 37 et Saf 84 (voir exemple 1)
   par immunométrie (figure 8) : capture par Saf 37 et révélation par un anticorps dirigé contre la région 94-230 de la PrP.

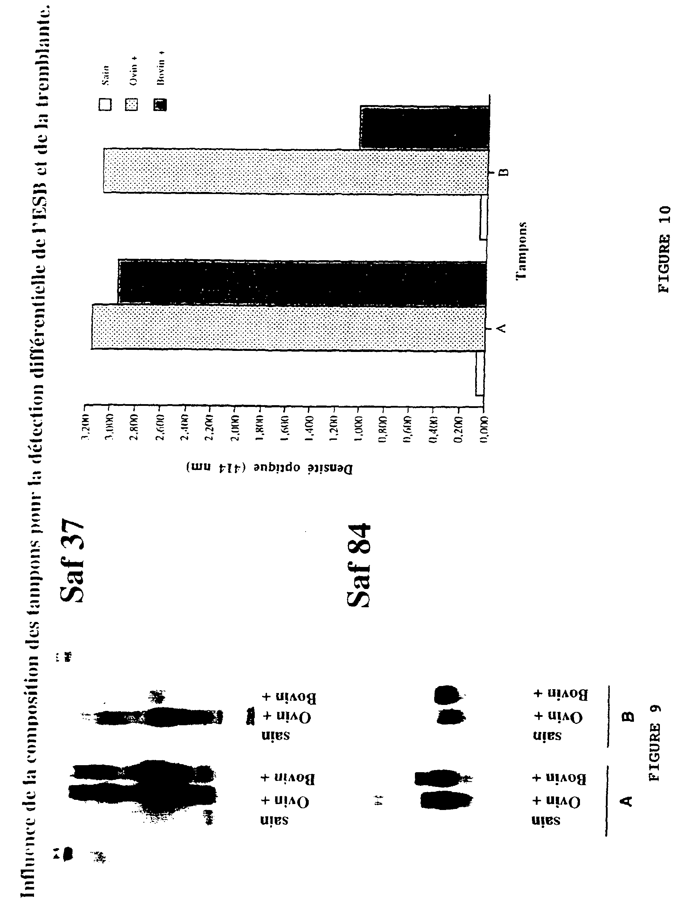

### - Figures 9 (Western blot) et 10 (dosage immunométrique à deux sites) :

. La comparaison est effectuée à partir d'homogénats à 20 % de cerveaux de mouton sain, de mouton atteint de tremblante et de bovin atteint d'ESB, obtenus, dans les conditions exposées à l'exemple 2.
. Traitement des échantillons :
   - **A :**: sarkosyl 10 %+Triton^{®} 10 %+urée 2M+protéinase K 60 µg/ml, 10 minutes
   - **B :**: SDS 10 %+Triton^{®} 5 %+urée 2M+protéinase K 180 µg/ml, 10 minutes
. Détection
   dans les mêmes conditions que ci-dessus.

L'augmentation seule de la dose en PK permet de faire apparaître une sensibilité différentielle de la région des motifs octa-peptide répétés, entre l'ESB et la tremblante chez le mouton mais pas chez l'homme (entre le type 1 et le type 4).

En revanche, en modifiant également la composition en agent tensioactif et en agent chaotrope cela permet de faire apparaître une sensibilité différentielle de la région des motifs octa-peptide répétés dans tous les cas.

### EXEMPLE 5 : Influence de la composition des tampons sur la détection différentielle de l'ESB et de la tremblante

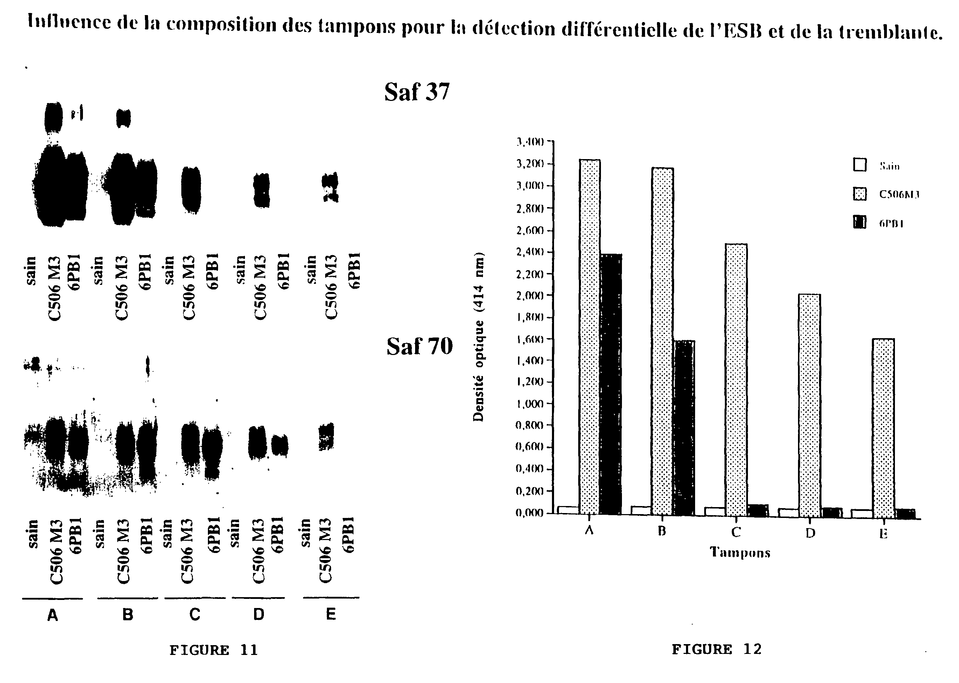

### - Figures 11 (Western blot) et 12 (dosage immunométrique à deux sites) :

- La comparaison est effectuée à partir d'homogénats à 10 % de cerveaux de souris saines ou de souris expérimentalement infectées avec la souche C506M3 (tremblante) ou avec une souche 6PB1 (ESB), obtenus, dans les conditions exposées à l'exemple 2.
- Traitement des échantillons :
   - **A :**: sarkosyl 10 %+Triton^{®} 10 %+urée 2M+protéinase K 30 µg/ml, 10 minutes
   - **B :**: sarkosyl 10 %+Triton^{®} 10 %+urée 2M+protéinase K 60 µg/ml, 10 minutes
   - **C :**: sarkosyl 10 %+Triton^{®} 10 %+urée 2M+protéinase K 180 µg/ml, 10 minutes
   - **D :**: sarkosyl 10 %+Triton^{®} 10 %+urée 2M+protéinase K 360 µg/ml, 10 minutes
   - **E :**: sarkosyl 10 %+urée 2M+protéinase K 180 µg/ml, 10 minutes.
- Détection
   par Western blot (figure 11) : anticorps Saf 37 et Saf 70 (voir exemple 1)
   par immunométrie (figure 12) : capture par Saf 37 et révélation par un anticorps dirigé contre la région 94-230 de la PrP.

Les résultats obtenus montrent que l'augmentation seule de la dose en PK permet de faire apparaître une sensibilité différentielle de la région des motifs octa-peptide répétés entre l'ESB et la tremblante, chez la souris.

### EXEMPLE 6 : Influence des tampons et de la concentration en protéinase K sur la détection des différentes MCJ.

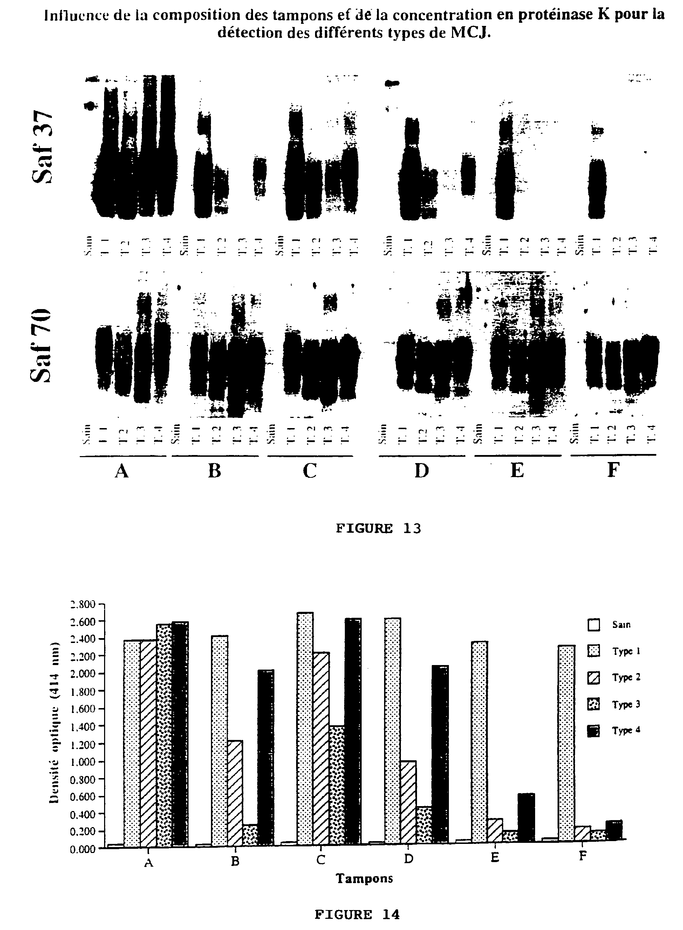

### - Figures 13 (Western blot) et 14 (dosage immunométrique à deux sites) :

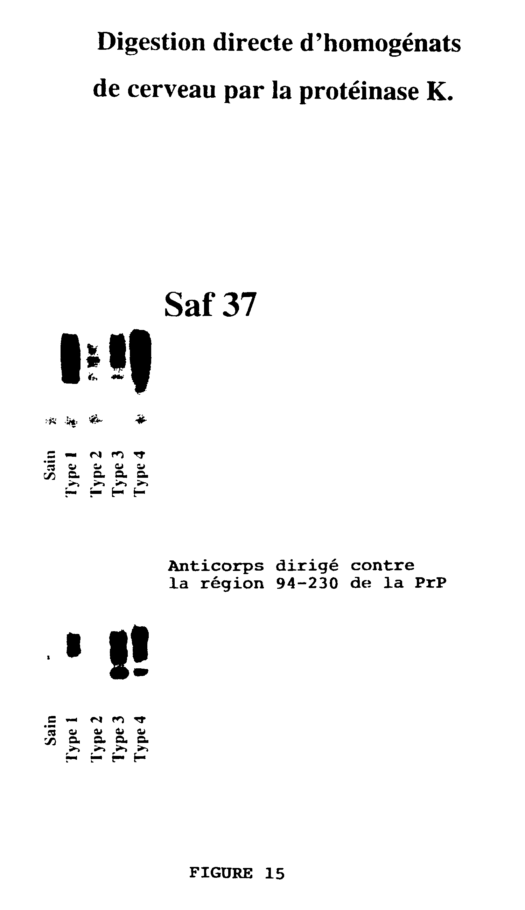

- La comparaison est effectuée à partir d'homogénats à 10 % de cerveaux d'hommes sains et d'hommes atteints de MCJ (types 1, 2, 3 et 4), obtenus dans les conditions exposées à l'exemple 2.
- Traitement des échantillons :
   - **A :**: sarkosyl 10 %+Triton^{®} 10 %+urée 2M+protéinase K 30 µg/ml, 10 minutes
   - **B :**: sarkosyl 10 %+Triton^{®} 10 %+urée 2M+protéinase K 180 µg/ml, 10 minutes
   - **C :**: sarkosyl 10 %+protéinase K 30 µg/ml, 10 minutes
   - **D :**: sarkosyl 10 %+protéinase K 60 µg/ml, 10 minutes
   - **E :**: sarkosyl 10 %+protéinase K 180 µg/ml, 10 minutes
   - **F :**: sarkosyl 10 %+protéinase K 360 µg/ml, 10 minutes.
- Détection
   par Western blot (figure 13) : anticorps Saf 37 et Saf 70 (voir exemple 1)
   par immunométrie (figure 14) : capture par Saf 37 et révélation par un anticorps dirigé contre la région 94-230 de la PrP.

### - Figures 15 (Western blot) et 16 (Immunométrie)

. La comparaison est effectuée à partir d'homogénats à 10 % de cerveaux d'hommes sains et d'hommes atteints de MCJ (types 1, 2, 3 et 4), obtenus dans les conditions exposées à l'exemple 2.
. Traitement des échantillons :
   Figure 15 : digestion par de la protéinase K (150 µg/ml), 10 minutes.

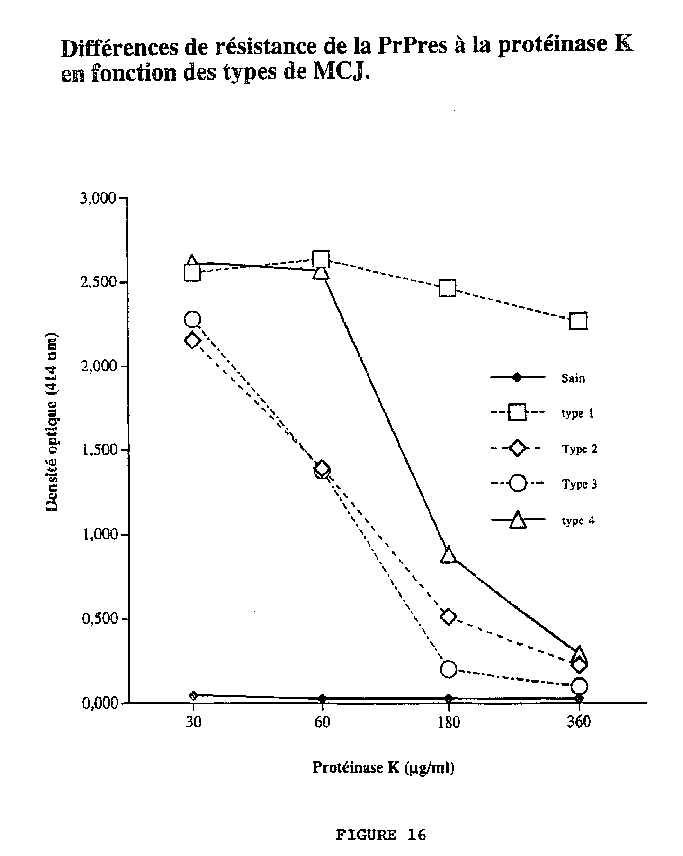

   Figure 16 : sarkosyl 10 %+urée 2M+protéinase K (de 30 à 360 µg/ml, pendant 10 minutes.
. Détection
   par Western blot (figure 15) : anticorps Saf 37 et un anticorps dirigé contre la région 94-230 de la PrP (voir exemple 1)
   par immunométrie (figure 16) : capture par Saf 37 et révélation par un anticorps dirigé contre la région 94-230 de la PrP.

Les résultats obtenus montrent que l'augmentation de la dose en PK permet de faire apparaître une sensibilité différentielle de la région des motifs octa-peptide répétés dans les différents types de MCJ. Dans ces conditions, il n'existe pas de différence significative entre le type 1 et le type 4 ; le fait de changer la composition en agent tensioactif et en agent chaotrope, à la même dose de PK (E, figures 13 et 14) permet de détruire les octa-peptides dans le type 4 tout en les conservant dans le type 1.

De plus, la figure 16 permet de montrer la différence de sensibilité des PrPres issues de différentes souches, pour un même tampon, en fonction de la dose en PK.

Par ailleurs, la figure 15 montre qu'un traitement direct de l'homogénat par de la PK met en évidence un autre type de sensibilité de la PrPres à la dégradation.

### EXEMPLE 7 : Détection par Western-blot de la PrPres digérée et purifiée sous forme de SAF.

Des homogénats, obtenus dans les conditions selon l'exemple 2, sont traités comme suit :
Homogénat 20 % (500 µl) + NaCl 20% (500 µl)+ [sarkosyl 20 % + SB314 2 %] (500 µl)+ PK (20 µg/ml final) pendant 1 heure.

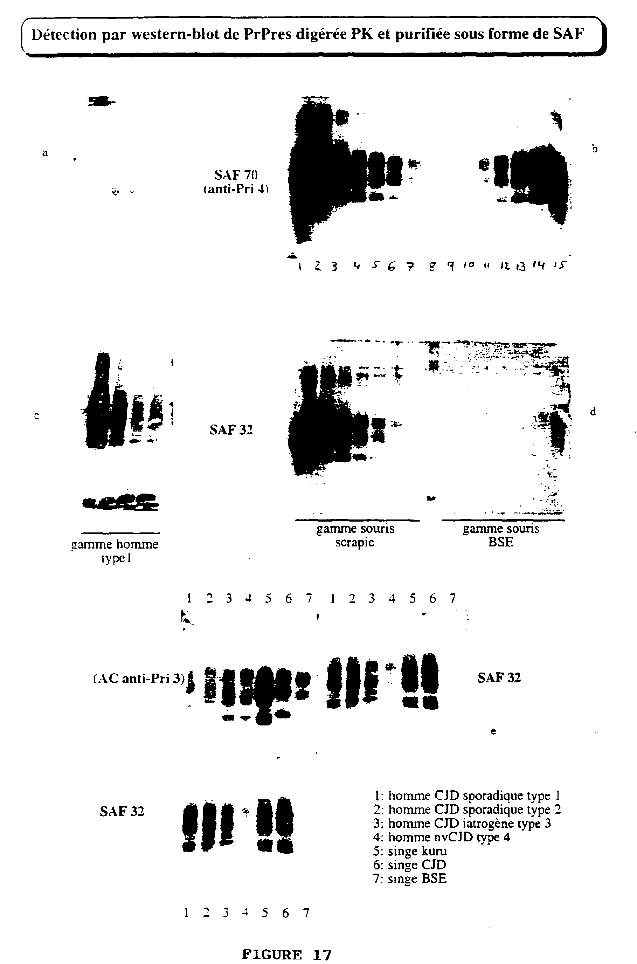

La figure 17 illustre les résultats :
   b et d:
      pistes 1-7 : résultats obtenus avec différentes dilutions de souche C506M3 de la tremblante chez la souris (dilutions 1/20, 1/50, 1/250, 1/500, 1/1000 et 1/2000)
      piste 8 : poids moléculaires
      pistes 9-15 : résultats obtenus avec différentes dilutions de souche EBS chez la souris (dilutions de 1/1000 à 1/10).

      Il est possible d'éliminer entièrement le signal ESB.
   a et c : les résultats obtenus confirment qu'il y a une augmentation significative du signal en présence d'anticorps dirigés contre les motifs octa-peptide répétés.
   e : cette figure montre qu'il est possible d'obtenir une diminution du signal avec ESB aussi bien chez le singe que chez l'homme (pistes 4 et 7).

### Références

. Butler D., *Nature,* 1998, 395, 6-7.
. Collinge J. et al., *Nature,* 1996, 393, 685-690.
. Créminon, C. et al., *J. Immunol. Methods,* 1993, 162, 179-192.
. Ellman, G. et al, *Biochem. Pharmacol.,* 1961, **7**, 88-95.
. Frobert, Y. et al., *Methods Mol. Biol.,* 1991, **80,** 57-68.
. Grassi, J. et al., *Anal. Biochem.,* 1988, **168,** 436-450.
. Grassi J. et al., *J. Immunol. Methods,* 1989, **123,** 193-210.
. Grathwohl K.U. et al., J. Virol. Methods, 1997, **64,** 205-216.
. Kuczius T. et al., *Mol. Med.,* 1999, **5,** 406-418.
. Lasmezas C. et al., *Nature,* 1996, **381,** 743-744.
. Lasmezas C.et al. *Science,* 1997, **275,** 402-405.
. McLaughlin L.L. et al., *Biochem. Biophys. Res. Comm.,* 1987, **144,** 469-476.
. Oesch B. et al., *Curr. Topics Microbiol. and Immunol.,* 1991, **172,** 109-124
. Oesch B. et al., *Biochemistry,* 1994, **33,** 5926-5931.
. Parchi P. et al., *Nature,* 1997, 386, 232-234.
. Priola S.A., *Nature Med.,* 1996, 2, 12, 1303-1304.
. Prusiner S.B. et al, *Cell,* 1984, 38, 127-134.
. Safar J. et al., *Nature Med.,* 1998, **10,** 1157-1165.
. Schaller O. et al. Acta Neuropathol., 1999, 98, 437-443.
. Serban D. et al., *Neurology,* 1990, **40**, 110.

## Revendications

1. Procédé de diagnostic d'une ESST ou maladie à prions provoquée par une souche d'ATNC par détection de la PrPres dans un échantillon biologique, **caractérisé en ce qu'**il comprend :
(1) le traitement dudit échantillon avec au moins une protéinase K, soit à une concentration comprise entre 30 µg/ml et 200 µg/ml pendant 10 minutes à 37°C pour un homogénat à 10 %, soit pendant une période et à une concentration équivalentes à la concentration comprise entre 30 µg/ml et 200 µg/ml dans les conditions précitées et de manière encore plus préférée pendant 10 minutes à une concentration comprise entre 30 µg/ml et 200 µg/ml pour un homogénat à 10 % ou pendant 30 minutes à une concentration comprise entre 10 µg/ml et 70 µg/ml, pour un homogénat à 10 %, ladite protéinase K étant mise en solution dans un tampon sélectionné dans le groupe constitué par les tampons d'homogénéisation de l'échantillon biologique et des tampons comprenant au moins l'un des agents suivants : au moins un agent tensioactif et/ou au moins un agent chaotrope et/ou au moins un sel, de manière à dégrader complètement la PrPsens, tout en conservant tout ou partie des motifs octa-peptide répétés de la PrPres, quelle que soit la souche d'ATNC,
(2) la mise en contact dudit échantillon traité avec un ligand desdits motifs octa-peptide et
(3) la détection de la présence éventuelle du complexe motifs octa-peptide répétés-ligand.

2. Procédé de diagnostic différentiel des ESST provoquées par des souches d'ATNC, dans un échantillon biologique, par détection des PrPres associées aux différentes souches d'ATNC, **caractérisé en ce qu'**il comprend :
(a) la détection de la PrPres d'une souche d'ATNC dans une première fraction dudit échantillon, conformément aux étapes (1) à (3) suivantes :
(1) le traitement dudit échantillon avec au moins une protéinase K, de manière à dégrader complètement la PrPsens, tout en conservant tout ou partie des motifs octa-peptide répétés de la PrPres, quelle que soit la souche d'ATNC,
(2) la mise en contact dudit échantillon traité avec un ligand desdits motifs octa-peptide et
(3) la détection de la présence éventuelle du complexe motifs octa-peptide répétés-ligand, puis :
(b) pour chaque échantillon pour lequel la présence d'un complexe motifs octa-peptide répétés-ligand est détectée à l'étape (a) :
- le traitement d'une deuxième fraction dudit échantillon avec au moins une protéinase K, de manière à ce que la majorité des motifs octa-peptide répétés soit éliminée pour la PrPres associée à au moins une souche d'intérêt, notamment la souche de l'ESB et où l'ensemble des PrPres associées aux autres souches d'ATNC conservent tout ou partie desdits motifs octa-peptide répétés,
- la mise en contact de ladite deuxième fraction dudit échantillon traité, avec un ligand desdits motifs octa-peptide répétés et
- la détection de la présence éventuelle du complexe motifs octa-peptide répétés-ligand.

3. Procédé de diagnostic différentiel des ESST provoquées par des souches d'ATNC dans un échantillon biologique considéré comme contenant de la PrPres, **caractérisé en ce qu'**il comprend pour chaque échantillon pour lequel la présence d'une PrPres a été détectée, la mise en oeuvre de l'étape (b) selon la revendication 2,
- la mise en contact de ladite deuxième fraction dudit échantillon traité, avec un ligand desdits motifs octa-peptide répétés, et
- la détection de la présence éventuelle du complexe motifs octa-peptide répétés-ligand.

4. Procédé selon la revendication 2 ou la revendication 3, **caractérisé en ce que** le traitement avec ladite protéinase K selon l'étape (a) ou (b) est réalisé pendant 30 secondes à 2 heures, à une température inférieure à 80°C, de préférence entre 10 minutes et 30 minutes.

5. Procédé selon la revendication 4, **caractérisé en ce que** le traitement avec ladite protéinase K est réalisé soit à une concentration comprise entre 30 µg/ml et 200 µg/ml pendant 10 minutes à 37°C pour un homogénat à 10 %, soit pendant une période et à une concentration équivalentes à la concentration comprise entre 30 µg/ml et 200 µg/ml dans les conditions précitées et de manière encore plus préférée pendant 10 minutes à une concentration comprise entre 30 µg/ml et 200 µg/ml pour un homogénat à 10 % ou pendant 30 minutes à une concentration comprise entre 10 µg/ml et 70 µg/ml, pour un homogénat à 10%.

6. Procédé selon l'une quelconque des revendications 2 à 5, **caractérisé en ce que** la protéinase K est mise en solution dans un tampon sélectionné dans le groupe constitué par les tampons d'homogénéisation de l'échantillon biologique et des tampons comprenant au moins l'un des agents suivants : au moins un agent tensioactif et/ou au moins un agent chaotrope et/ou au moins un sel.

7. Procédé selon la revendication 1 ou la revendication 6, **caractérisé en ce que** ledit tampon comprend de préférence :
**a. au moins un agent tensioactif,** sélectionné dans le groupe constitué par :
- des tensioactifs anioniques, tels que le SDS (dodécylsulfate de sodium), le sarkosyl (lauroyl-sarcosine), le cholate de sodium, le désoxycholate de sodium, le taurocholate de sodium ;
- des tensioactifs zwitterioniques, tels que le SB 3-10 (décyl-sulfobétaïne), le SB 3-12 (dodécyl-sulfobétaïne), le SB 3-14, le SB 3-16 (hexadécyl-sulfobétaïne), le CHAPS et le désoxyCHAPS ;
- des tensioactifs non-ioniques, tels que le C12E8 (dodécyl-octa-éthylèneglycol), le TRITON X100, le TRITON X114, le TWEEN 20, le TWEEN 80, le MEGA 9 (nonanoyl-méthylglucamine), l'octylglucoside, le LDAO (dodécyl-diméthylamine oxyde) ou le NP40 ou
- des mélanges de tensioactifs tels qu'un mélange d'un agent tensioactif ionique et d'un agent tensioactif non-ionique, un mélange de deux agents tensioactifs ioniques ou un mélange d'un agent tensioactif ionique et d'un agent tensioactif zwitterionique et/ou
**b. au moins un agent chaotrope** sélectionné dans le groupe constitué par l'urée et la guanidine ou un mélange de ceux-ci et/ou
**c**. au moins un sel sélectionné parmi les sels de métaux alcalins ou non.

8. Procédé selon la revendication 7, **caractérisé en ce que** ledit tampon comprend au moins 5% de tensioactif anionique, de préférence du sarkosyl, éventuellement associé à du SDS.

9. Procédé selon l'une quelconque des revendications 1 à 8, **caractérisé en ce que** le ligand est sélectionné dans le groupe constitué par des aptamères et des anticorps qui se lient à la région des motifs octapeptide répétés.

10. Procédé selon la revendication 2, la revendication 4 ou la revendication 5, **caractérisé en ce que** le traitement de l'étape (b) est réalisé dans les mêmes conditions que celles définies à l'étape (a) (1), mais à une concentration en PK supérieure à celle utilisée à l'étape (a) (1).

11. Trousse de diagnostic pour la mise en oeuvre des procédés selon l'une quelconque des revendications 1 à 10, **caractérisée en ce qu'**elle comprend en association au moins un agent tensioactif et/ou au moins un agent chaotrope et /ou au moins un sel et une protéase tels que définis à l'une quelconque des revendications 1 à 10.

## Claims

1. Method for diagnosing a TSSE or prion disease caused by a UTA strain, by detecting PrPres in a biological sample, **characterized in that** it comprises:
(1) treating said sample with at least one proteinase K, either at a concentration of between 30 µg/ml and 200 µg/ml for 10 minutes at 37°C for a homogenate at 10%, or for a period and at a concentration which are equivalent to the concentration of between 30 µg/ml and 200 µg/ml under the abovementioned conditions, and even more preferably for 10 minutes at a concentration of between 30 µg/ml and 200 µg/ml for a homogenate at 10% or for 30 minutes at a concentration of between 10 µg/ml and 70 µg/ml for a homogenate at 10%, **said proteinase K being** dissolved in a buffer selected from the group consisting of the biological sample homogenization buffers and buffers comprising at least one of the following agents: at least one surfactant and/or at least one chaotropic agent and/or at least one salt, in such a way as to completely degrade PrPsens, while at the same time conserving all or some of the octapeptide motif repeats of PrPres, whatever the UTA strain,
(2) bringing said treated sample into contact with a ligand of said octapeptide motifs, and
(3) detecting the possible presence of the octapeptide motif repeats/ligand complex.

2. Method for the differential diagnosis of TSSEs caused by UTA strains, in a biological sample, by detecting the PrPres associated with the various UTA strains, **characterized in that** it comprises:
(a) detecting PrPres of a UTA strain in a first fraction of said sample, in accordance with the following steps (1) to (3):
(1) treating said sample with at least one proteinase K, in such a way as to completely degrade PrPsens, while at the same time conserving all or some of the octapeptide motif repeats of PrPres, whatever the UTA strain,
(2) bringing said treated sample into contact with a ligand of said octapeptide motifs, and
(3) detecting the possible presence of the octapeptide motif repeats/ligand complex.
(b) for each sample for which the presence of an octapeptide motif repeats/ligand complex is detected in step (a):
- treating a second fraction of said sample with at least one proteinase K, in such a way that the majority of the octapeptide motif repeats are eliminated for the PrPres associated with at least one strain of interest, in particular the BSE strain, and such that all of the PrPres associated with the other UTA strains conserve all or some of said octapeptide motif repeats,
- bringing said second fraction of said sample treated into contact with a ligand of said octapeptide motif repeats, and
- detecting the possible presence of the octapeptide motif repeats/ligand complex.

3. Method for the differential diagnosis of TSSEs caused by UTA strains, in a biological sample considered to contain PrPres, **characterized in that** it comprises, for each sample for which the presence of a PrPres has been detected, carrying out step (b) as claimed in claim 2,
- bringing said second fraction of said sample treated into contact with a ligand of said octapeptide motif repeats, and
- detecting the possible presence of the octapeptide motif repeats/ligand complex.

4. Method as claimed in claim 2 or claim 3, **characterized in that** the treatment with said proteinase K according to steps(a) or (b) is carried out for 30 seconds to 2 hours, at a temperature of less than 80°C, preferably between 10 minutes and 30 minutes.

5. Method as claimed in claim 4, **characterized in that** the treatment with said proteinase K is carried out either at a concentration of between 30 µg/ml and 200 µg/ml for 10 minutes at 37°C for a homogenate at 10%, or for a period and at a concentration which are equivalent to the concentration of between 30 µg/ml and 200 µg/ml under the abovementioned conditions, and even more preferably for 10 minutes at a concentration of between 30 µg/ml and 200 µg/ml for a homogenate at 10% or for 30 minutes at a concentration of between 10 µg/ml and 70 µg/ml for a homogenate at 10%.

6. Method as claimed in any one of claims 2 to 5, **characterized in that** the proteinase K is dissolved in a buffer selected from the group consisting of the biological sample homogenization buffers and buffers comprising at least one of the following agents: at least one surfactant and/or at least one chaotropic agent and/or at least one salt.

7. Method as claimed in claim 1 or claim 6, **characterized in that** said buffer preferably comprises:
**a. at least one surfactant,** selected from the group consisting of:
- anionic surfactants, such as SDS (sodium dodecyl sulfate), sarkosyl (lauroylsarcosine), sodium cholate, sodium deoxycholate or sodium taurocholate;
- zwitterionic surfactants, such as SB 3-10 (decylsulfobetaine), SB 3-12 (dodecylsulfobetaine), SB 3-14, SB 3-16 (hexadecylsulfobetaine), CHAPS and deoxyCHAPS;
- nonionic surfactants, such as C12E8 (dodecyloctaethylene glycol), TRITON X100, TRITON X114, TWEEN 20, TWEEN 80, MEGA 9 (nonanoylmethylglucamine), octylglucoside, LDAO (dodecyldimethylamine oxide) or NP40, or
- mixtures of surfactants, such as a mixture of an ionic surfactant and a nonionic surfactant, a mixture of two ionic surfactants or a mixture of an ionic surfactant and a zwitterionic surfactant, and/or
**b. at least one chaotropic agent,** selected from the group consisting of urea and guanidine, or a mixture thereof, and/or
**c. at least one salt** selected from the salts of metals which may or may not be alkali metals.

8. Method as claimed in claim 7, **characterized in that** said buffer comprises at least 5% of anionic surfactant, preferably sarkosyl, optionally combined with SDS.

9. Method as claimed in any one of claims 1 to 8, **characterized in that** the ligand is selected from the group consisting of aptamers and antibodies capable of binding to the region of the octapeptide motif repeats.

10. Method as claimed in claim 2, claim 4 or claim 5, **characterized in that** the treatment of step (b) is carried out under the same conditions as those defined in step (a) (1), but at a concentration of PK higher than that used in step (a) (1).

11. Diagnostic kit for carrying out the methods as claimed in any one of claims 1 to 10, **characterized in that** it comprises, in combination, at least one surfactant and/or at least one chaotropic agent and/or at least one salt and a protease, as defined in any one of claims 1 to 10.

## Patentansprüche

1. Diagnostisches Verfahren für eine von einem ATNC-Stamm verursachte ESST oder Prionenerkrankung durch Nachweis des PrPres in einer biologischen Probe, **dadurch gekennzeichnet, dass** es umfasst:
(1) Behandlung der Probe mit wenigstens einer Proteinase K, entweder in einer Konzentration zwischen 30 µg/ml und 200 µg/ml für 10 Minuten bei 37 °C für ein 10%iges Homogenat oder für eine Dauer und bei einer Konzentration entsprechend der Konzentration zwischen 30 µg/ml und 200 µg/ml unter den oben genannten Bedingungen, und besonders bevorzugt für 10 Minuten bei einer Konzentration zwischen 30 µg/ml und 200 µg/ml für ein 10%iges Homogenat oder für 30 Minuten bei einer Konzentration zwischen 10 µg/ml und 70 µg/ml für ein 10%iges Homogenat, wobei die Proteinase K in Lösung in einem Puffer eingesetzt wird, der ausgewählt ist aus der Gruppe bestehend aus Homogenisierungspuffern für die biologische Probe und Puffern, die wenigstens eines der folgenden Mittel umfassen: wenigstens ein Tensid und/oder wenigstens ein chaotropes Mittel und/oder wenigstens ein Salz, so dass das PrPsens vollständig abgebaut wird, während die Octapeptidmotiv-Wiederholungen des PrPres unabhängig davon, um welchen ATNC-Stamm es sich handelt, ganz oder teilweise erhalten bleiben,
(2) In-Kontakt-Bringen der behandelten Probe mit einem Liganden für die Octapeptidmotive, und
(3) Nachweis des eventuellen Vorliegens des Komplexes aus Octapeptidmotiv-Wiederholungen-Ligand.

2. Differentialdiagnostisches Verfahren für von ATNC-Stämmen verursachte ESSTs durch Nachweis der mit den verschiedenen ATNC-Stämmen assoziierten PrPres in einer biologischen Probe, **dadurch gekennzeichnet, dass** es umfasst:
(a) Nachweis des PrPres von einem ATNC-Stamm in einer ersten Fraktion der Probe gemäß den folgenden Schritten (1) bis (3):
(1) Behandlung der Probe mit wenigstens einer Proteinase K, so dass das PrPsens vollständig abgebaut wird, während die Octapeptidmotiv-Wiederholungen des PrPres unabhängig davon, um welchen ATNC-Stamm es sich handelt, ganz oder teilweise erhalten bleiben,
(2) In-Kontakt-Bringen der behandelten Probe mit einem Liganden für die Octapeptidmotive, und
(3) Nachweis des eventuellen Vorliegens des Komplexes aus Octapeptidmotiv-Wiederholungen-Ligand, und dann:
(b) für jede Probe, für die das Vorliegen eines Komplexes aus Octapeptidmotiv-Wiederholungen-Ligand in Schritt (a) nachgewiesen wird:
- Behandlung einer zweiten Fraktion der Probe mit wenigstens einer Proteinase K, so dass der größte Teil der Octapeptidmotiv-Wiederholungen für das mit wenigstens einem Stamm von Interesse, insbesondere dem ESB-Stamm, assoziierte PrPres abgebaut wird, und die Gruppe der mit den anderen ATNC-Stämmen assoziierten PrPres die Octapeptidmotiv-Wiederholungen ganz oder teilweise behält,
- In-Kontakt-Bringen der zweiten Fraktion der behandelten Probe mit einem Liganden für die Octapeptidmotiv-Wiederholungen, und
- Nachweis des eventuellen Vorliegens des Komplexes aus Octapeptidmotiv-Wiederholungen-Ligand.

3. Differentialdiagnostisches Verfahren für von ATNC-Stämmen verursachte ESSTs in einer biologischen Probe, von der man annimmt, dass sie PrPres enthält, **dadurch gekennzeichnet, dass** es für jede Probe, für die das Vorliegen eines PrPres nachgewiesen wurde, die Durchführung von Schritt (b) nach Anspruch 2 umfasst,
- das In-Kontakt-Bringen der zweiten Fraktion der behandelten Probe mit einem Liganden für die Octapeptidmotiv-Wiederholungen, und
- den Nachweis des eventuellen Vorliegens des Komplexes aus Octapeptidmotiv-Wiederholungen-Ligand.

4. Verfahren nach Anspruch 2 oder Anspruch 3, **dadurch gekennzeichnet, dass** die Behandlung mit der Proteinase K nach Schritt (a) oder (b) 30 Sekunden bis 2 Stunden bei einer Temperatur unter 80 °C durchgeführt wird, vorzugsweise 10 Minuten bis 30 Minuten.

5. Verfahren nach Anspruch 4, **dadurch gekennzeichnet, dass** die Behandlung mit der Proteinase K entweder bei einer Konzentration zwischen 30 µg/ml und 200 µg/ml für 10 Minuten bei 37 °C für ein 10%iges Homogenat durchgeführt wird oder für eine Dauer und bei einer Konzentration entsprechend der Konzentration zwischen 30 µg/ml und 200 µg/ml unter den oben genannten Bedingungen, und besonders bevorzugt für 10 Minuten bei einer Konzentration zwischen 30 µg/ml und 200 µg/ml für ein 10%iges Homogenat oder für 30 Minuten bei einer Konzentration zwischen 10 µg/ml und 70 µg/ml für ein 10%iges Homogenat.

6. Verfahren nach irgendeinem der Ansprüche 2 bis 5, **dadurch gekennzeichnet, dass** die Proteinase K in Lösung in einem Puffer eingesetzt wird, der ausgewählt ist aus der Gruppe bestehend aus Homogenisierungspuffern für die biologische Probe und Puffern, die wenigstens eines der folgenden Mittel umfassen: wenigstens ein Tensid und/oder wenigstens ein chaotropes Mittel und/oder wenigstens ein Salz.

7. Verfahren nach Anspruch 1 oder Anspruch 6, **dadurch gekennzeichnet, dass** der Puffer vorzugsweise umfasst:
a. wenigstens ein Tensid, ausgewählt aus der Gruppe bestehend aus:
- anionischen Tensiden wie SDS (Natriumdodecylsulfat), Sarkosyl (Lauroylsarcosin), Natriumcholat, Natriumdesoxycholat, Natriumtaurocholat;
- zwitterionischen Tensiden wie SB 3-10 (Decyl-Sulfobetain), SB 3-12 (Dodecyl-Sulfobetain), SB 3-14, SB 3-16 (Hexadecyl-Sulfobetain), CHAPS und DeoxyCHAPS;
- nichtionischen Tensiden wie C12E8 (Dodecyloctaethylenglycol), TRITON X100, TRITON X114, TWEEN 20, TWEEN 80, MEGA 9 (Nonanoylmethylglucamin), Octylglucosid, LDAO (Dodecyldimethylaminoxid) oder NP40, oder
- Mischungen von Tensiden, wie eine Mischung aus einem ionischen Tensid und einem nichtionischen Tensid, eine Mischung aus zwei ionischen Tensiden oder eine Mischung aus einem ionischen Tensid und einem zwitterionischen Tensid, und/oder
b. wenigstens ein chaotropes Mittel, ausgewählt aus der Gruppe bestehend aus Harnstoff und Guanidin oder einer Mischung davon, und/oder
c. wenigstens ein Salz, ausgewählt aus Alkalimetall- oder Nichtalkalimetallsalzen.

8. Verfahren nach Anspruch 7, **dadurch gekennzeichnet, dass** der Puffer wenigstens 5% anionisches Tensid, vorzugsweise Sarkosyl, gegebenenfalls zusammen mit SDS umfasst.

9. Verfahren nach irgendeinem der Ansprüche 1 bis 8, **dadurch gekennzeichnet, dass** der Ligand ausgewählt ist aus der Gruppe bestehend aus Aptameren und Antikörpern, die an die Region der Octapeptidmotiv-Wiederholungen binden.

10. Verfahren nach Anspruch 2, Anspruch 4 oder Anspruch 5, **dadurch gekennzeichnet, dass** die Behandlung von Schritt (b) unter den gleichen Bedingungen durchgeführt wird, wie sie in Schritt (a)(1) definiert sind, aber bei einer PK-Konzentration über der, die in Schritt (a)(1) verwendet wird.

11. Diagnostischer Kit zur Durchführung des Verfahrens nach irgendeinem der Ansprüche 1 bis 10, **dadurch gekennzeichnet, dass** er in Kombination wenigstens ein Tensid und/oder wenigstens ein chaotropes Mittel und/oder wenigstens ein Salz und eine Protease enthält, wie definiert in irgendeinem der Ansprüche 1 bis 10.
